# EUROPEAN PATENT APPLICATION

(11) **EP 3 903 782 A1**
(43) Date of publication of application: **03.11.2021**
(21) Application number: 19906140.9
(22) Date of filing: 27.12.2019
(51) Int. Cl.: A61K 31/423, A61P 1/16, A61P 3/06, A61K 9/14, A61K 9/16, A61K 9/20, A61K 9/48, A61K 47/32, A61K 47/38

(54) **PHARMACEUTICAL COMPOSITION**

(30) Priority: 27.12.2018 JP 2018245700; 27.12.2018 JP 2018245706
(71) Applicant: KOWA COMPANY, LTD., Naka-ku Nagoya-shi Aichi 460-8625 (JP)
(72) Inventor: SUGIMOTO, Shin, Fuji-shi, Shizuoka 417-8650 (JP); MINAMIZONO, Akito, Fuji-shi, Shizuoka 417-8650 (JP); NISHIDA, Chisa, Fuji-shi, Shizuoka 417-8650 (JP)
(74) Representative: Blodig, Wolfgang
(86) International application number: PCT/JP2019/051389
(87) International publication number: WO 2020/138406

(57) **Abstract**

Provided is a pharmaceutical composition containing pemafibrate, a salt thereof or a solvate thereof and having excellent homogeneity. The pharmaceutical composition is provided to contain the following components (A) and (B): (A) pemafibrate, a salt thereof or a solvate thereof; and (B) one or more selected from the group consisting of the following components (B-1) and (B-7) : (B-1) an alkylcellulose and a salt thereof; and a (meth)acrylic acid-based polymer.

## Description

### Field of the Invention

The present invention relates to a pharmaceutical composition etc.

### Background of the Invention

It is known that pemafibrate (Chemical Name: (2R)-2-[3-([1,3-Benzoxazol-2-yl[3-(4-methoxyphenoxy)propyl] amino]methyl)phenoxy]butanoic acid)(International Nonproprietary Name: pemafibrate) represented by the following structural formula:

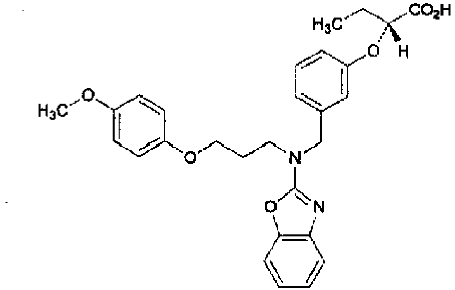

, a salt thereof or a solvate thereof has excellent PPAR-α agonist activity, exhibits plasma triglyceride concentration reducing action, HDL cholesterol increasing action, etc., and is useful for prevention and treatment of dyslipidemia (hyperlipidemia) (Patent Document 1 and Non-Patent Documents 1 and 2), and useful for prevention and treatment of NAFLD (non-alcoholic fatty liver disease) (Patent Document 2).

Meanwhile, a compound useful as an active component for a pharmaceutical preparation is normally formulated as some pharmaceutical composition and supplied, and from the viewpoint of reliably exhibiting expected drug efficacy and avoiding unanticipated adverse side effects, it is very important that the pharmaceutical composition to be supplied maintains a certain level of quality without variations such as lot-to-lot variation.

### Prior Art Documents

### Patent Documents

Patent Document 1: International Publication No. WO 2005/023777
Patent Document 2: International Publication No. WO 2015/005365

### Non-Patent Documents

Non-Patent Document 1: Yukiyoshi Yamazaki, et al., Synthesis, 2008(7), 1017-1022.
Non-Patent Document 2: Fruchart JC., Cardiovasc Diabetol., 2013; 12: 82.

### Summary of the Invention

### Problems to be Solved by the Invention

However, manufacturability of pharmaceutical compositions, such as homogeneity, significantly depends on the physical and chemical properties of components, but it is often impossible to predict such properties from the chemical structures of the components, and there are not a few cases where a problem becomes evident only when a pharmaceutical composition is actually produced. Thus, establishment of a technique for securing homogeneity of a pharmaceutical composition commonly requires considerable try and error.

Pemafibrate, a salt thereof or a solvate thereof has been only reported to exhibit the above-described pharmacological effects, and has heretofore not been specifically studied in terms of a pharmaceutical composition, and manufacturability such as homogeneity of the pharmaceutical composition has heretofore not been reported at all.

In these circumstances, for developing a pharmaceutical composition containing pemafibrate, a salt thereof or a solvate thereof, the present inventors have first actually produced the pharmaceutical composition. As a result, it was found that pharmaceutical compositions becomes different in content of pemafibrate, leading to development of problems with homogeneity (uniformity) of the content of pemafibrate. If pharmaceutical compositions significantly differ in content of pemafibrate, there may be variations in efficacy and safety among the pharmaceutical compositions.

Thus, an object of the present invention is to provide a pharmaceutical composition containing pemafibrate, a salt thereof or a solvate thereof, and having excellent homogeneity.

### Means for Solving the Problems

In order to solve the problem with the content uniformity of pemafibrate, a salt thereof or a solvate thereof in a pharmaceutical composition, the present inventors have further extensively conducted studies, and found that by further incorporating any of the following components 1 to 7 (hereinafter, components 1 to 7 are sometimes referred to as "component (B-1)" , "component (B-2)", "component (B-3)", "component (B-4)", "component (B-5)", "component (B-6)" and "component (B-7)", respectively, and "one or more selected from the group consisting of components (B-1) to (B-7)" is sometimes referred to as "component (B)"):

1. cellulose ether species typified by croscarmellose, carmellose, hydroxypropylcellulose, hydroxypropylmethylcellulose, methylcellulose and ethylcellulose;
2. starch species typified by pregelatinized starch, corn starch and carboxymethyl starch sodium;
3. povidone species typified by crospovidone and polyvinyl pyrrolidone;
4. silicic acid compound typified by hydrous magnesium silicate, hydrated silicon dioxide and light anhydrous silicic acid;
5. polyhydric alcohol typified by macrogol and mannitol;
6. alkyl sulfate ester typified by sodium lauryl sulfate; and
7. (meth)acrylic acid-based polymers typified by aminoalkyl methacrylate copolymer E, ammonioalkyl methacrylate copolymer, dried methacrylic acid copolymer LD, methacrylic acid copolymer S and methacrylic acid copolymer L, in a pharmaceutical composition comprising pemafibrate, a salt thereof or a solvate thereof (hereinafter, sometimes referred to simply as "component (A)"), the content uniformity of pemafibrate in the pharmaceutical composition is improved. The present invention has been accomplished on the basis of this finding.

Accordingly, the present invention provides a pharmaceutical composition comprising the following components (A) and (B):
(A) pemafibrate, a salt thereof or a solvate thereof; and
(B) one or more selected from the group consisting of the following components (B-1) to (B-7):
   (B-1) a cellulose ether species;
   (B-2) a starch species;
   (B-3) a povidone species;
   (B-4) a silicic acid compound;
   (B-5) a polyhydric alcohol;
   (B-6) an alkyl sulfate ester; and
   (B-7) a (meth)acrylic acid-based polymer.

The present invention also provides a method for improving the content uniformity of pemafibrate, a salt thereof or a solvate thereof in a pharmaceutical composition, the method including the step of incorporating one or more selected from the group consisting of components (B-1) to (B-7) in a pharmaceutical composition containing pemafibrate, a salt thereof or a solvate thereof.

### Effects of the Invention

According to the present invention, it is possible to provide a pharmaceutical composition having improved content uniformity of pemafibrate in the pharmaceutical composition and having excellent homogeneity.

### Detailed Description of the Invention

### <Pemafibrate, salt thereof or solvate thereof (Component (A))>

Herein, "pemafibrate, a salt thereof or a solvate thereof" includes pemafibrate (Chemical Name: (2R)-2-[3-([1,3-Benzoxazol-2-yl[3-(4-methoxyphenoxy)propyl] amino]methyl)phenoxy]butanoic acid) (International Nonproprietary Name: pemafibrate) itself, a pharmaceutically acceptable salt of pemafibrate and a solvate of pemafibrate or a pharmaceutically acceptable salt thereof with water, alcohol (for example ethanol) or the like. The pharmaceutically acceptable salt is not particularly limited, and examples thereof include acid addition salts and base addition salts. Specific examples of the acid addition salts include acid addition salts with inorganic acids, such as hydrochlorides, hydrobromides, hydroiodides, sulfate salts, nitrate salts and phosphate salts; and acid addition salts with organic acids, such as benzoate salts, methanesulfonate salts, ethanesulfonate salts, benzenesulfonate salts, p-toluenesulfonate salts, maleate salts, fumarate salts, tartrate salts, citrate salts and acetate salts. Specific examples of the base addition salts include metal salts such as sodium salts, potassium salts, lithium salts, calcium salts and magnesium salts; salts with amines such as ammonia, trimethylamine, triethylamine, pyridine, collidine and lutidine; and base addition salts with organic bases such as lysine, arginine, cinchonine and cinchonidine.

The shape, the size and the like of pemafibrate, a salt thereof or a solvate thereof are not particularly limited, and when the average particle diameter of primary particles is measured in accordance with The Japanese Pharmacopoeia, 17th Edition, Laser Diffraction Measurement of Particle Size, d50 and d90 values are preferably as follows.
d50: preferably 100 µm or less, more preferably 50 µm or less, still more preferably 20 µm or less, particularly preferably 1 to 20 µm.
d90: preferably 200 µm or less, more preferably 135 µm or less, still more preferably 80 µm or less, particularly preferably 1 to 80 µm.

Pemafibrate, a salt thereof or a solvate thereof is a known compound, and can be produced through a method as disclosed in Patent Document 1, Non-Patent Document 1 or U.S. Patent No. 7,109,226, for example. In the present invention, a pemafibrate crystal which can be produced through the method described in Non-Patent Document 1 (preferably a crystal having a melting point of 95 to 101°C, particularly preferably 97 to 100°C in measurement performed in accordance with The Japanese Pharmacopoeia, 17th Edition, Melting Point Determination Method 1) is preferably used. The disclosures of the documents are incorporated herein by reference.

The content of pemafibrate, a salt thereof or a solvate thereof in the pharmaceutical composition is not particularly limited, and can be determined in appropriate consideration of the target disease, the type of preparation, the sex, age and symptoms of a patient in need of the composition, and the like. For example, the content can be set so that the daily dose of pemafibrate, a salt thereof or a solvate thereof may be 0.05 to 0.8 mg, more preferably 0.075 to 0.6 mg, particularly preferably 0.1 to 0.4 mg, in terms of a free form of pemafibrate.

The content of pemafibrate, a salt thereof or a solvate thereof in the pharmaceutical composition is preferably 0.01 to 5 mass%, more preferably 0.025 to 1 mass%, particularly preferably 0.05 to 0.5 mass%, in terms of a free form of pemafibrate, with respect to the total mass of the pharmaceutical composition. According to the present invention, even if pemafibrate, a salt thereof or a solvate thereof has such a small content, a good content uniformity can be obtained.

### <Cellulose ether species (Component (B-1))>

Herein, the "cellulose ether species" means one or more selected from the group consisting of a compound in which all or some of hydroxy groups of cellulose form ether bonds; and a salt thereof. The cellulose ether species may be cellulose to which in addition to etherification, further modification such as esterification or crosslink formation as necessary has been applied. Here, the salt is not particularly limited, and specific examples thereof include alkali metal salts such as sodium salts and potassium salts; and salts with metals of Group 2 elements, such as calcium salts and magnesium salts. The average degree of polymerization, the form (crystal form) and the like of the cellulose ether species are not particularly limited, and the average degree of polymerization is preferably 10 to 10,000.

Specific examples of the cellulose ether species include alkylcelluloses or salts thereof such as methylcellulose and ethylcellulose; hydroxyalkylcelluloses or salts thereof such as hydroxyethylcellulose and hydroxypropylcellulose; alkyl(hydroxyalkyl)celluloses, derivatives (ester derivatives) thereof or salts thereof such as hydroxyethylmethylcellulose, hypromellose, hypromellose acetate succinate ester and hypromellose phthalate ester; and carboxyalkylcelluloses, derivatives (cross-linked polymers) thereof or salts thereof such as carmellose, carmellose potassium, carmellose calcium, carmellose sodium, carboxymethylethylcellulose and croscarmellose sodium, and these cellulose ethers may be used singly, or in combinations of two or more thereof. The alkyl group in the cellulose ether species is not particularly limited, and is preferably a linear or branched C1-C6 alkyl group. The degree of substitution with hydroxyalkoxy groups in the hydroxyalkylcellulose is not particularly limited, and for example, hydroxypropylcellulose includes both non-low substituted hydroxypropylcellulose and low substituted hydroxypropylcellulose. Here, the low substituted hydroxypropylcellulose refers to hydroxypropylcellulose in which the hydroxypropoxy group content determined in a dried state is 5.0 to 16.0% as described in The Japanese Pharmacopoeia, 17th Edition.

From the viewpoint of improvement of content uniformity, the cellulose ether species is preferably one or more selected from the group consisting of an alkylcellulose, a hydroxyalkylcellulose, an alkyl(hydroxyalkyl)cellulose, a carboxyalkylcellulose, a cross-linked polymer of a carboxyalkylcellulose and a salt thereof, more preferably one or more selected from the group consisting of a C1-C6 alkylcellulose, a hydroxy C1-C6 alkylcellulose, a C1-C6 alkyl(hydroxy C1-C6 alkyl)cellulose, a carboxy C1-C6 alkylcellulose, a cross-linked polymer of a carboxy C1-C6 alkylcellulose and a salt thereof, still more preferably one or more selected from the group consisting of methylcellulose, ethylcellulose, hydroxypropylcellulose, hypromellose, carmellose, croscarmellose and a salt thereof, yet more preferably one or more selected from the group consisting of methylcellulose, ethylcellulose, hydroxypropylcellulose, hypromellose, carmellose, carmellose potassium, carmellose calcium, carmellose sodium and croscarmellose sodium, still further preferably one or more selected from the group consisting of methylcellulose, ethylcellulose, hydroxypropylcellulose, hypromellose, carmellose, carmellose calcium, carmellose sodium and croscarmellose sodium, particularly preferably ethylcellulose. The hydroxypropylcellulose is preferably low substituted hydroxypropylcellulose. From the viewpoint of ease of production of a pharmaceutical composition (particularly a solid preparation), the cellulose ether species is preferably solid at normal temperature (any temperature in the range of 15 to 25°C) .

The rate of alkoxy group substitution in alkylcellulose or a salt thereof is preferably within a range of from 20 to 70%, more preferably within a range of from 40 to 60%. Determination of the rate of alkoxy group substitution in alkylcellulose or a salt thereof is performed according to the determination method for methoxy group in a methylcellulose, as described in the Japanese Pharmacopoeia, 17th Edition. Additionally, alkylcellulose or a salt thereof preferably has a viscosity of from 0.1 to 5000 mPa·s, more preferably from 1 to 200 mPa·s. Measurement of the viscosity of alkylcellulose or a salt thereof is carried out according to the viscosity measurement method for methylcellulose, as described in the Japanese Pharmacopoeia, 17th Edition.

Each of these cellulose ether species is a known component. The cellulose ether species may be produced through a known method, or commercially available products may be used. Examples of the commercially available products include ETHOCEL (Dow Chemical Japan Limited), CMEC (Freund Corporation), NS-300 (San-Ei Gen F.F.I., Inc.), ECG-505 (San-Ei Gen F.F.I., Inc.), CELLOGEN (San-Ei Gen F.F.I., Inc.), Ac-Di-Sol (Asahi Kasei Corporation), HEC (Sumitomo Seika Chemicals Co., Ltd.), Hydroxypropylcellulose (Nippon Soda Co., Ltd.), Shin-Etsu AQOAT (Shin-Etsu Chemical Co., Ltd.), METOLOSE 90SH-SR (Shin-Etsu Chemical Co., Ltd.), HPMCP (Shin-Etsu Chemical Co., Ltd.), METOLOSE SM (Shin-Etsu Chemical Co., Ltd.), TC-5 (San-Ei Gen F.F.I., Inc.) and L-HPC (Shin-Etsu Chemical Co., Ltd.).

The content of the cellulose ether species in the pharmaceutical composition is not particularly limited, and can be determined in appropriate consideration of the type of preparation, the sex, age and symptoms of a patient in need of the composition, and the like, but from the viewpoint of improvement of content uniformity, the total amount of the cellulose ether species with respect to the total mass of the pharmaceutical composition is preferably 0.5 to 30 mass%, more preferably 1 to 20 mass%, still more preferably 1.5 to 15 mass%, particularly preferably 2 to 10 mass%.

When alkylcelluloses or salts thereof are used as cellulose ether species, the content of the alkylcelluloses or salts thereof with respect to the total mass of the pharmaceutical composition is preferably 0.6 to 22 mass%, more preferably 1.1 to 19 mass%, particularly preferably 2 to 8 mass%, from the viewpoint of improvement of content uniformity.

When hydroxyalkylcelluloses or salts thereof are used as cellulose ether species, the content of the hydroxyalkylcelluloses or salts thereof with respect to the total mass of the pharmaceutical composition is preferably 0.7 to 24 mass%, more preferably 1.2 to 18 mass%, particularly preferably 3 to 8 mass%, from the viewpoint of improvement of content uniformity.

When alkyl(hydroxyalkyl)celluloses, derivatives thereof or salts thereof are used as cellulose ether species, the content of the alkyl(hydroxyalkyl)celluloses, derivatives thereof or salts thereof with respect to the total mass of the pharmaceutical composition is preferably 0.8 to 26 mass%, more preferably 1.3 to 17 mass%, particularly preferably 4 to 9 mass%, from the viewpoint of improvement of content uniformity.

When carboxyalkylcelluloses, derivatives thereof or salts thereof are used as cellulose ether species, the content of the carboxyalkylcelluloses, derivatives thereof or salts thereof with respect to the total mass of the pharmaceutical composition is preferably 0.9 to 28 mass%, more preferably 1.4 to 16 mass%, particularly preferably 1.6 to 9 mass%, from the viewpoint of improvement of content uniformity.

The mass ratio between the content of pemafibrate, a salt thereof or a solvate thereof and the content of the cellulose ether species in the pharmaceutical composition is not particularly limited, and from the viewpoint of improvement of content uniformity, the total content of the cellulose ether species with respect to 1 part by mass of a free form of pemafibrate is preferably 3 to 200 parts by mass, more preferably 5 to 150 parts by mass, particularly preferably 10 to 100 parts by mass.

When alkylcelluloses or salts thereof are used as cellulose ether species, the mass ratio between the content of pemafibrate, a salt thereof or a solvate thereof and the content of the alkylcelluloses or salts thereof in the pharmaceutical composition is not particularly limited, and from the viewpoint of improvement of content uniformity, the total content of the alkylcelluloses or salts thereof with respect to 1 part by mass of a free form of pemafibrate is preferably 4 to 160 parts by mass, more preferably 6 to 110 parts by mass, particularly preferably 20 to 60 parts by mass.

When hydroxyalkylcelluloses or salts thereof are used as cellulose ether species, the mass ratio between the content of pemafibrate, a salt thereof or a solvate thereof and the content of the hydroxyalkylcelluloses or salts thereof in the pharmaceutical composition is not particularly limited, and from the viewpoint of improvement of content uniformity, the total content of the hydroxyalkylcelluloses or salts thereof with respect to 1 part by mass of a free form of pemafibrate is preferably 4 to 170 parts by mass, more preferably 7 to 120 parts by mass, still more preferably 20 to 100 parts by mass, particularly preferably 30 to 70 parts by mass.

When alkyl(hydroxyalkyl)celluloses, derivatives thereof or salts thereof are used as cellulose ether species, the mass ratio between the content of pemafibrate, a salt thereof or a solvate thereof and the content of the alkyl(hydroxyalkyl)celluloses, derivatives thereof or salts thereof in the pharmaceutical composition is not particularly limited, and from the viewpoint of improvement of content uniformity, the total content of the alkyl(hydroxyalkyl)celluloses, derivatives thereof or salts thereof with respect to 1 part by mass of a free form of pemafibrate is preferably 4 to 180 parts by mass, more preferably 8 to 130 parts by mass, still more preferably 20 to 100 parts by mass, particularly preferably 40 to 80 parts by mass.

When carboxyalkylcelluloses, derivatives thereof or salts thereof are used as cellulose ether species, the mass ratio between the content of pemafibrate, a salt thereof or a solvate thereof and the content of the carboxyalkylcelluloses, derivatives thereof or salts thereof in the pharmaceutical composition is not particularly limited, and from the viewpoint of improvement of content uniformity, the total content of the carboxyalkylcelluloses, derivatives thereof or salts thereof with respect to 1 part by mass of a free form of pemafibrate is preferably 4 to 190 parts by mass, more preferably 9 to 140 parts by mass, still more preferably 14 to 100 parts by mass, particularly preferably 19 to 90 parts by mass.

### <Starch species (Component (B-2))>

Herein, the "starch species" means one or more selected from the group consisting of starch itself; starch in which all or some of hydroxy groups form ether bonds; a derivative thereof; and a salt thereof. The starch species include those subjected to treatment such as gelatinization or aging. The derivative includes starch or etherified products thereof in which further modification such as esterification, crosslink formation or hydrolysis is applied. Here, the salt is not particularly limited, and specific examples thereof include alkali metal salts such as sodium salts and potassium salts; and salts with metals of Group 2 elements, such as calcium salts and magnesium salts.

Specific examples of the starch species include starches or salts thereof such as pregelatinized starch, wheat starch, rice starch, corn starch, potato starch, partially pregelatinized starch, wheat flour, rice flour and semi-digested starch; hydroxyalkyl ethers of starch or salts thereof such as hydroxypropyl starch; and carboxyalkyl ethers of starch or salts thereof such as carboxymethyl starch sodium, and these starches may be used singly, or in combinations of two or more thereof. The alkyl group in the starch species is not particularly limited, and is preferably a linear or branched C1-C6 alkyl group.

From the viewpoint of improvement of content uniformity, the starch species is preferably one or more selected from the group consisting of starch, a hydroxyalkyl ether of starch, a carboxyalkyl ether of starch and a salt thereof, more preferably one or more selected from the group consisting of starch, a hydroxy C1-C6 alkyl ether of starch, a carboxy C1-C6 alkyl ether and a salt thereof, still more preferably one or more selected from the group consisting of starch, hydroxypropyl starch, carboxymethyl starch and a salt thereof, particularly preferably one or more selected from the group consisting of starch and carboxymethyl starch sodium. From the viewpoint of ease of production of a pharmaceutical composition (particularly a solid preparation), the starch species is preferably solid at normal temperature (any temperature in the range of 15 to 25°C) .

Each of these starch species is a known component. The starch species may be produced through a known method, or commercially available products may be used. Examples of the commercially available products include LYCATAB PGS (Roquette Japan K.K.), GLYCOLYS (Roquette Japan K.K.), Starch (soluble) (Kishida Chemical Co., Ltd.), Corn Starch (San-Ei Gen F.F.I., Inc.), Potato Starch (JUNSEI CHEMICAL CO., LTD.), HPS-101 (Freund Corporation) and LYCATABC (Roquette Japan K.K.).

The content of the starch species in the pharmaceutical composition is not particularly limited, and can be determined in appropriate consideration of the type of preparation, the sex, age and symptoms of a patient in need of the composition, and the like, but from the viewpoint of improvement of content uniformity, the total amount of the starch species with respect to the total mass of the pharmaceutical composition is preferably 0.5 to 50 mass%, more preferably 1 to 40 mass%, still more preferably 1.5 to 30 mass%, particularly preferably 2 to 20 mass%.

When starch is used as starch species, the content of the starch with respect to the total mass of the pharmaceutical composition is preferably 0.6 to 47 mass%, more preferably 1.1 to 38 mass%, particularly preferably 1.6 to 28 mass%, from the viewpoint of improvement of content uniformity.

When carboxyalkyl ethers of starch or salts thereof are used as starch species, the content of the carboxyalkyl ethers of starch or salts thereof with respect to the total mass of the pharmaceutical composition is preferably 0.8 to 45 mass%, more preferably 1.3 to 36 mass%, particularly preferably 1.7 to 26 mass%, from the viewpoint of improvement of content uniformity.

The mass ratio between the content of pemafibrate, a salt thereof or a solvate thereof and the content of the starch species in the pharmaceutical composition is not particularly limited, and from the viewpoint of improvement of content uniformity, the total content of the starch species with respect to 1 part by mass of a free form of pemafibrate is preferably 5 to 400 parts by mass, more preferably 15 to 300 parts by mass, particularly preferably 20 to 200 parts by mass.

When starch is used as starch species, the mass ratio between the content of pemafibrate, a salt thereof or a solvate thereof and the content of the starch in the pharmaceutical composition is not particularly limited, and from the viewpoint of improvement of content uniformity, the total content of the starch with respect to 1 part by mass of a free form of pemafibrate is preferably 7 to 380 parts by mass, more preferably 16 to 280 parts by mass, particularly preferably 30 to 190 parts by mass.

When carboxyalkyl ethers of starch or salts thereof are used as starch species, the mass ratio between the content of pemafibrate, a salt thereof or a solvate thereof and the content of the carboxyalkyl ethers of starch or salts thereof in the pharmaceutical composition is not particularly limited, and from the viewpoint of improvement of content uniformity, the total content of the carboxyalkyl ethers of starch or salts thereof with respect to 1 part by mass of a free form of pemafibrate is preferably 9 to 370 parts by mass, more preferably 17 to 270 parts by mass, particularly preferably 40 to 180 parts by mass.

### <Povidone species (Component (B-3))>

Herein, the "povidone species" means polymers of 1-vinyl-2-pyrrolidone, and includes not only homopolymers of 1-vinyl-2-pyrrolidone but also copolymers of 1-vinyl-2-pyrrolidone and other polymerizable compounds. The polymer may be either a non-cross-linked polymer or a cross-linked polymer.

The K value of a linear-chain polymer of 1-vinyl-2-pyrrolidone (povidone) is not particularly limited; the indicated K value is preferably 12 to 90, particularly preferably 25 to 90.

Specific examples of the povidone species include linear-chain polymers of 1-vinyl-2-pyrrolidone, such as povidone (the K value of the povidone is not particularly limited, and the indicated K value is, for example, 12, 17, 25, 30 or 90); copolymers of 1-vinyl-2-pyrrolidone and vinyl acetate, such as copolyvidone; and cross-linked polymers of 1-vinyl-2-pyrrolidone, such as crospovidone. These povidones may be used singly, or in combinations of two or more thereof.

From the viewpoint of improvement of content uniformity, the povidone species is preferably one or more selected from the group consisting of povidone, copolyvidone and crospovidone, more preferably one or more selected from the group consisting of povidone and crospovidone, particularly preferably crospovidone. From the viewpoint of ease of production of a pharmaceutical composition (particularly a solid preparation), the povidone species is preferably solid at normal temperature (any temperature in the range of 15 to 25°C).

Each of these povidone species is a known component. The povidone species may be produced through a known method, or commercially available products may be used. Examples of the commercially available products include Kollidon CL, Kollidon VA64 and Kollidon (each from BASF Japan Ltd.).

The content of the povidone species in the pharmaceutical composition is not particularly limited, and can be determined in appropriate consideration of the type of preparation, the sex, age and symptoms of a patient in need of the composition, and the like, but from the viewpoint of improvement of content uniformity, the total amount of the povidone species with respect to the total mass of the pharmaceutical composition is preferably 0.1 to 20 mass%, more preferably 0.5 to 15 mass%, particularly preferably 1 to 10 mass%.

When linear-chain polymers of 1-vinyl-2-pyrrolidone are used as povidone species, the content of the linear-chain polymers of 1-vinyl-2-pyrrolidone with respect to the total mass of the pharmaceutical composition is preferably 0.2 to 16 mass%, more preferably 0.6 to 14 mass%, particularly preferably 3 to 9 mass%, from the viewpoint of improvement of content uniformity.

When cross-linked polymers of 1-vinyl-2-pyrrolidone are used as povidone species, the content of the cross-linked polymers of 1-vinyl-2-pyrrolidone with respect to the total mass of the pharmaceutical composition is preferably 0.3 to 17 mass%, more preferably 0.7 to 13 mass%, particularly preferably 2 to 8 mass%, from the viewpoint of improvement of content uniformity.

The mass ratio between the content of pemafibrate, a salt thereof or a solvate thereof and the content of the povidone species in the pharmaceutical composition is not particularly limited, and from the viewpoint of improvement of content uniformity, the total content of the povidone species with respect to 1 part by mass of a free form of pemafibrate is preferably 1 to 200 parts by mass, more preferably 3 to 150 parts by mass, particularly preferably 5 to 100 parts by mass.

When linear-chain polymers of 1-vinyl-2-pyrrolidone are used as povidone species, the mass ratio between the content of pemafibrate, a salt thereof or a solvate thereof and the content of the linear-chain polymers of 1-vinyl-2-pyrrolidone is not particularly limited, and from the viewpoint of improvement of content uniformity, the total content of the linear-chain polymers of 1-vinyl-2-pyrrolidone with respect to 1 part by mass of a free form of pemafibrate is preferably 1.5 to 190 parts by mass, more preferably 3.5 to 140 parts by mass, particularly preferably 6 to 90 parts by mass.

When cross-linked polymers of 1-vinyl-2-pyrrolidone are used as povidone species, the mass ratio between the content of pemafibrate, a salt thereof or a solvate thereof and the content of the cross-linked polymers of 1-vinyl-2-pyrrolidone is not particularly limited, and from the viewpoint of improvement of content uniformity, the total content of the cross-linked polymers of 1-vinyl-2-pyrrolidone with respect to 1 part by mass of a free form of pemafibrate is preferably 2 to 180 parts by mass, more preferably 4 to 130 parts by mass, particularly preferably 7 to 80 parts by mass.

### <Silicic acid compound (Component (B-4))>

Herein, the "silicic acid compound" includes silicic acid compounds themselves, and salts of silicic acid compounds. Examples of the salts of silicic acid compounds include inorganic salts, and specific examples thereof include alkali metal salts such as sodium salts and potassium salts; salts with metals of Group 2 elements, such as magnesium salts and calcium salts; and salts with metals of Group 13 elements, such as aluminum salts.

Specific examples of the silicic acid compounds include hydrous silicic acid compounds or salts thereof such as hydrated silicon dioxide, amorphous silicon oxide hydrate, hydrous magnesium silicate and hydrous magnesium silicate (natural); anhydrous silicic acids or salts thereof such as light anhydrous silicic acid and heavy anhydrous silicic acid; silicic acids or salts thereof such as silicon dioxide, natural aluminum silicate, synthetic aluminum silicate, synthetic sodium magnesium silicate, calcium silicate, magnesium silicate, aluminum magnesium silicate, magnesium aluminosilicate and magnesium aluminometasilicate; diatomaceous earth; bentonite; kaolin; and talc, and these compounds may be used singly, or in combinations of two or more thereof.

From the viewpoint of improvement of content uniformity, the silicic acid compound is preferably one or more selected from the group consisting of a hydrous silicic acid compound, a salt of a hydrous silicic acid compound, hydrous silicic acid and a salt of hydrous silicic acid, particularly preferably one or more selected from the group consisting of a hydrous silicic acid compound and a salt of a hydrous silicic acid compound.

Among the silicic acid compounds shown as examples, one or more selected from the group consisting of hydrous magnesium silicate, hydrated silicon dioxide and light anhydrous silicic acid are preferable, and one or more selected from the group consisting of hydrous magnesium silicate and hydrated silicon dioxide are more preferable, from the viewpoint of improvement of content uniformity. From the viewpoint of ease of production of a pharmaceutical composition (particularly a solid preparation), the silicic acid compound is preferably solid at normal temperature (any temperature in the range of 15 to 25°C) .

Each of these silicic acid compounds is a known component. The silicic acid compounds may be produced through a known method, or commercially available products may be used. Examples of the commercially available products include Neusilin A (Fuji Chemical Industries Co., Ltd.), FLORITE (Tomita Pharmaceutical Co., Ltd.), Magnesium Silicate (Tomita Pharmaceutical Co., Ltd.), VEEGUMI GRANULE (Sanyo Chemical Industries, Ltd.), VEEGUMI HV GRANULE (Sanyo Chemical Industries, Ltd.), VEEGUMI K GRANULE (Sanyo Chemical Industries, Ltd.), VEEGUMI F (Sanyo Chemical Industries, Ltd.), SYLYSIA 320 (FUJI SILYSIA CHEMICAL LTD.), SYLYSIA 350 (FUJI SILYSIA CHEMICAL LTD.), SYLYSIA 320TP (FUJI SILYSIA CHEMICAL LTD.), SYLYSIA 320FCP (FUJI SILYSIA CHEMICAL LTD.), MICON FR (Tomita Pharmaceutical Co., Ltd.), Silicon Dioxide (NIPPON AEROSIL CO., LTD.), AEROSIL 300 (NIPPON AEROSIL CO., LTD.), Adsolider 101 (Freund Corporation), Adsolider 102 (Freund Corporation), SYLYSIA (FUJI SILYSIA CHEMICAL LTD.), SYLOSPHERE (FUJI SILYSIA CHEMICAL LTD.), Hydrous Amorphous Silicon Oxide (Tosoh Silica Corporation), Neusilin (Fuji Chemical Industries Co., Ltd.), Diatomaceous Earth (Showa Kako Corporation) and Talc (San-Ei Gen F.F.I., Inc.).

The content of the silicic acid compounds in the pharmaceutical composition is not particularly limited, and can be determined in appropriate consideration of the type of preparation, the sex, age and symptoms of a patient in need of the composition, and the like, but from the viewpoint of improvement of content uniformity, the total amount of the silicic acid compounds with respect to the total mass of the pharmaceutical composition is preferably 0.1 to 20 mass%, more preferably 0.5 to 15 mass%, particularly preferably 1 to 10 mass%.

When one or more selected from the group consisting of a hydrous silicic acid compound and a salt thereof are used as silicic acid compounds, the content of one or more selected from the group consisting of a hydrous silicic acid compound and a salt thereof with respect to the total mass of the pharmaceutical composition is preferably 0.2 to 19 mass%, more preferably 0.6 to 14 mass%, particularly preferably 2 to 6 mass%, from the viewpoint of improvement of content uniformity.

When one or more selected from the group consisting of an anhydrous silicic acid compound and a salt thereof are used as silicic acid compounds, the content of one or more selected from the group consisting of am anhydrous silicic acid compound and a salt thereof with respect to the total mass of the pharmaceutical composition is preferably 0.4 to 17 mass%, more preferably 0.8 to 12 mass%, particularly preferably 4 to 8 mass%, from the viewpoint of improvement of content uniformity.

The mass ratio between the content of pemafibrate, a salt thereof or a solvate thereof and the content of the silicic acid compounds in the pharmaceutical composition is not particularly limited, and from the viewpoint of improvement of content uniformity, the total content of the silicic acid compounds with respect to 1 part by mass of a free form of pemafibrate is preferably 1 to 200 parts by mass, more preferably 3 to 150 parts by mass, particularly preferably 5 to 100 parts by mass.

When one or more selected from the group consisting of a hydrous silicic acid compound and a salt thereof are used as silicic acid compounds, the mass ratio between the content of pemafibrate, a salt thereof or a solvate thereof and the content of one or more selected from the group consisting of a hydrous silicic acid compound and a salt thereof is not particularly limited, and from the viewpoint of improvement of content uniformity, the total content of one or more selected from the group consisting of a hydrous silicic acid compound and a salt thereof with respect to 1 part by mass of a free form of pemafibrate is preferably 2 to 160 parts by mass, more preferably 4 to 140 parts by mass, particularly preferably 10 to 90 parts by mass.

When one or more selected from the group consisting of an anhydrous silicic acid compound and a salt thereof are used as silicic acid compounds, the mass ratio between the content of pemafibrate, a salt thereof or a solvate thereof and the content of one or more selected from the group consisting of an anhydrous silicic acid compound and a salt thereof is not particularly limited, and from the viewpoint of improvement of content uniformity, the total content of one or more selected from the group consisting of an anhydrous silicic acid compound and a salt thereof with respect to 1 part by mass of a free form of pemafibrate is preferably 2 to 180 parts by mass, more preferably 4 to 120 parts by mass, particularly preferably 10 to 80 parts by mass.

### <Polyhydric alcohol (Component (B-5))>

Herein, the "polyhydric alcohol" means compounds not having two or more cyclic ether structures (for example tetrahydropyran rings) in the molecule while having two or more alcoholic hydroxyl groups, and it may be either a non-polymer or a polymer. Examples of the polyhydric alcohol include sugar alcohols and non-sugar alcohols, and these alcohols may be used singly, or in combinations of two or more thereof. The polyhydric alcohol is preferably a polyhydric alcohol having no cyclic ether structure (for example tetrahydropyran ring) in the molecule or a polyhydric alcohol having only one cyclic ether structure in the molecule, more preferably a polyhydric alcohol having no cyclic ether structure in the molecule, particularly preferably a polyhydric alcohol which is an acyclic compound.

From the viewpoint of ease of production of a pharmaceutical composition (particularly a solid preparation), the polyhydric alcohol is preferably solid at normal temperature (any temperature in the range of 15 to 25°C) .

Specific examples of the sugar alcohol include C3 sugar alcohols (tritols) such as glycerin; C4 sugar alcohols (tetritols) such as erythritol and threitol; C5 sugar alcohols (pentitols) such as xylitol, arabinitol, ribitol and adonitol; C6 sugar alcohols (hexitols) such as mannitol, sorbitol, iditol, dulcitol and galactitol; and C12 sugar alcohols (dodecitols), such as maltitol and lactitol, and these sugar alcohols may be used singly, or in combinations of two or more thereof. For these sugar alcohols, various stereoisomers may be present. The steric configuration of the "sugar alcohol" is not particularly limited, and the "sugar alcohol" may be present as a single stereoisomer, or as a mixture of various stereoisomers at any ratio.

From the viewpoint of improvement of content uniformity, the sugar alcohol is preferably one or more selected from the group consisting of erythritol, xylitol, mannitol, sorbitol, maltitol and lactitol, more preferably one or more selected from the group consisting of mannitol, sorbitol and maltitol, particularly preferably mannitol.

Each of these sugar alcohols is a known component. The sugar alcohols may be produced through a known method, or commercially available products may be used. Examples of the commercially available products include Erythritol (San-Ei Gen F.F.I., Inc.), Xylit (Towa Chemical Industry Co., Ltd.), NEOSORB P (Roquette Japan K.K.), Lesys (Towa Chemical Industry Co., Ltd.), Mannit P (Towa Chemical Industry Co., Ltd.), Glycerin (NOF CORPORATION), MALTISORB (Roquette Japan K.K.) and Amalty Syrup (Towa Chemical Industry Co., Ltd.)

The non-sugar alcohol is preferably a non-sugar alcohol which is an acyclic compound. Specific examples thereof include alkylene glycols such as ethylene glycol, propylene glycol, 1,3-propanediol, 2-methyl-1,3-propanediol and 1,3-butanediol; polyalkylene glycols such as diethylene glycol, dipropylene glycol, macrogol (for example macrogol 100, macrogol 200, macrogol 300, macrogol 400, macrogol 600, macrogol 1000, macrogol 1500, macrogol 1540, macrogol 4000, macrogol 6000, polyethylene glycol 8000, macrogol 20000 and macrogol 35000), polypropylene glycol (for example polypropylene glycol 2000), polyoxyethylene polyoxypropylene glycol (for example polyoxyethylene (3) polyoxypropylene (17) glycol, polyoxyethylene (20) polyoxypropylene (20) glycol, polyoxyethylene (42) polyoxypropylene (67) glycol, polyoxyethylene (54) polyoxypropylene (39) glycol, polyoxyethylene (105) polyoxypropylene (5) glycol, polyoxyethylene (120) polyoxypropylene (40) glycol, polyoxyethylene (124) polyoxypropylene (39) glycol, polyoxyethylene (160) polyoxypropylene (30) glycol, polyoxyethylene (196) polyoxypropylene (67) glycol and polyoxyethylene (200) polyoxypropylene (70) glycol; polyvinyl alcohols such as (fully saponified) polyvinyl alcohol and (partially saponified) polyvinyl alcohol; and meglumine, and these non-sugar alcohols may be used singly, or in combinations of two or more thereof.

From the viewpoint of improvement of content uniformity, the non-sugar alcohol is preferably a dihydric non-sugar alcohol, more preferably a polyalkylene glycol, still more preferably macrogol, yet more preferably one or more selected from the group consisting of macrogol 100, macrogol 200, macrogol 300, macrogol 400, macrogol 600, macrogol 1000, macrogol 1500, macrogol 1540, macrogol 4000, macrogol 6000, polyethylene glycol 8000, macrogol 20000 and macrogol 35000, yet more preferably macrogol having an average molecular weight of 100 to 10,000, yet more preferably macrogol having an average molecular weight of 200 to 8,000, particularly preferably macrogol 6000. The average molecular weight of macrogol can be measured in accordance with "Average molecular mass" described in The Japanese Pharmacopoeia, 17th Edition, Pharmaceutical Preparations, Macrogol 400.

Each of these non-sugar alcohols is a known component. The non-sugar alcohols may be produced through a known method, or commercially available products may be used. Examples of the commercially available products include Kollisolv PG (BASF Japan Ltd.), Diethylene Glycol (Nippon Shokubai Co., Ltd.), UNISAFE DPG-R (NOF CORPORATION), Macrogol 200 (Sanyo Chemical Industries, Ltd.), Kollisolv PEG300 (BASF Japan Ltd.), SUPER REFINED PEG 400 (Croda Japan K.K.), CARBOWAX Sentry PEG 600 (Dow Chemical Japan Limited), Macrogol 1000 (NOF CORPORATION), Macrogol 1500 (Sanyo Chemical Industries, Ltd.), CARBOWAX Sentry PEG 1540 (Dow Chemical Japan Limited), Macrogol 4000 (Sanyo Chemical Industries, Ltd.), Macrogol 6000 (Sanyo Chemical Industries, Ltd.), Macrogol 20000 (Sanyo Chemical Industries, Ltd.), NEWPOL PP-2000 (Sanyo Chemical Industries, Ltd.), PRONON 101P (NOF CORPORATION), Kollisolv P124 (BASF Japan Ltd.), PRONON 403P (NOF CORPORATION), NEWDET PE-85 (Sanyo Chemical Industries, Ltd.), PEP-101 (Freund Corporation), Kolliphor P188 (BASF Japan Ltd.), Kolliphor P407 Micro (BASF Japan Ltd.) and UNILUBE DP-950B (NOF CORPORATION).

The content of the polyhydric alcohols in the pharmaceutical composition is not particularly limited, and can be determined in appropriate consideration of the type of preparation, the sex, age and symptoms of a patient in need of the composition, and the like, but from the viewpoint of improvement of content uniformity, the total amount of the polyhydric alcohols with respect to the total mass of the pharmaceutical composition is preferably 0.1 to 99 mass%, more preferably 0.5 to 95 mass%, still more preferably 1 to 90 mass%, particularly preferably 1.5 to 50 mass%.

When sugar alcohols are used as polyhydric alcohols, the content of the sugar alcohols with respect to the total mass of the pharmaceutical composition is preferably 0.2 to 98 mass%, more preferably 0.6 to 94 mass%, particularly preferably 1.1 to 85 mass%, from the viewpoint of improvement of content uniformity.

When non-sugar alcohols are used as polyhydric alcohols, the content of the non-sugar alcohols with respect to the total mass of the pharmaceutical composition is preferably 0.3 to 97 mass%, more preferably 0.7 to 93 mass%, particularly preferably 1.2 to 80 mass%, from the viewpoint of improvement of content uniformity.

When polyalkylene glycols are used as polyhydric alcohols, the content of the polyalkylene glycols with respect to the total mass of the pharmaceutical composition is preferably 0.4 to 96 mass%, more preferably 0.8 to 92 mass%, particularly preferably 1.3 to 75 mass%, from the viewpoint of improvement of content uniformity.

The mass ratio between the content of pemafibrate, a salt thereof or a solvate thereof and the content of the polyhydric alcohols in the pharmaceutical composition is not particularly limited, and from the viewpoint of improvement of content uniformity, the total content of the polyhydric alcohols with respect to 1 part by mass of a free form of pemafibrate is preferably 1 to 2,000 parts by mass, more preferably 5 to 1,500 parts by mass, still more preferably 10 to 1,000 parts by mass, particularly preferably 15 to 500 parts by mass.

When sugar alcohols are used as polyhydric alcohols, the mass ratio between the content of pemafibrate, a salt thereof or a solvate thereof and the content of the sugar alcohols in the pharmaceutical composition is not particularly limited, and from the viewpoint of improvement of content uniformity, the total content of the sugar alcohols with respect to 1 part by mass of a free form of pemafibrate is preferably 2 to 1,900 parts by mass, more preferably 6 to 1,450 parts by mass, particularly preferably 12 to 950 parts by mass.

When non-sugar alcohols are used as polyhydric alcohols, the mass ratio between the content of pemafibrate, a salt thereof or a solvate thereof and the content of the non-sugar alcohols in the pharmaceutical composition is not particularly limited, and from the viewpoint of improvement of content uniformity, the total content of the non-sugar alcohols with respect to 1 part by mass of a free form of pemafibrate is preferably 3 to 1,850 parts by mass, more preferably 7 to 1,400 parts by mass, particularly preferably 13 to 900 parts by mass.

When polyalkylene glycols are used as polyhydric alcohols, the mass ratio between the content of pemafibrate, a salt thereof or a solvate thereof and the content of the polyalkylene glycols in the pharmaceutical composition is not particularly limited, and from the viewpoint of improvement of content uniformity, the total content of the polyalkylene glycols with respect to 1 part by mass of a free form of pemafibrate is preferably 4 to 1,800 parts by mass, more preferably 8 to 1,350 parts by mass, particularly preferably 14 to 850 parts by mass.

### <Alkyl sulfate ester (Component (B-6))>

Herein, the "alkyl sulfate ester" means an alkyl sulfate ester salt represented by the following formula:

R-O-SO₃M ... (1)

[wherein R represents a linear or branched saturated or unsaturated C8-C22 hydrocarbon group, and M is an alkali metal such as sodium or potassium; a metal of a Group 2 element such as magnesium or calcium; an ammonium ion; or a C2 or C3 hydroxyalkyl-substituted ammonium such as triethanolammonium].

Specific examples of the alkyl sulfate ester include lauryl sulfate ester salts, tetradecyl sulfate ester salts, hexadecyl sulfate ester salts and octadecyl sulfate ester salts, and these sulfate ester salts may be used singly, or in combinations of two or more thereof.

From the viewpoint of improvement of content uniformity, the alkyl sulfate ester is preferably one or more selected from the group consisting of a lauryl sulfate ester salt, a tetradecyl sulfate ester salt, a hexadecyl sulfate ester salt and an octadecyl sulfate ester salt, more preferably a lauryl sulfate ester salt, particularly preferably sodium lauryl sulfate. From the viewpoint of ease of production of a pharmaceutical composition (particularly a solid preparation), the alkyl sulfate ester is preferably solid at normal temperature (any temperature in the range of 15 to 25°C).

Each of these alkyl sulfate esters is a known component. The alkyl sulfate esters may be produced through a known method, or commercially available products may be used. Examples of the commercially available products include Kolliphor SLS (BASF Japan Ltd.).

The content of the alkyl sulfate esters in the pharmaceutical composition is not particularly limited, and can be determined in appropriate consideration of the type of preparation, the sex, age and symptoms of a patient in need of the composition, and the like, but from the viewpoint of improvement of content uniformity, the total amount of the alkyl esters with respect to the total mass of the pharmaceutical composition is preferably 0.1 to 20 mass%, more preferably 0.5 to 15 mass%, particularly preferably 1 to 10 mass%.

The mass ratio between the content of pemafibrate, a salt thereof or a solvate thereof and the content of the alkyl sulfate esters in the pharmaceutical composition is not particularly limited, and from the viewpoint of improvement of content uniformity, the total content of the alkyl sulfate esters with respect to 1 part by mass of a free form of pemafibrate is preferably 1 to 200 parts by mass, more preferably 3 to 150 parts by mass, still more preferably 5 to 100 parts by mass, particularly preferably 5 to 50 parts by mass.

### <(Meth)acrylic acid-based polymers (Component (B-7))>

Herein, the term "(meth)acrylic acid-based polymers" means a polymer having one or more structural units selected from the group consisting of structural units derived from acrylic acid, structural units derived from methacrylic acid, structural units derived from acrylic acid esters and structural units derived from methacrylic acid esters (hereinafter, also referred to as structural unit X). Examples of acrylic acid esters and methacrylic acid esters include esters of (meth)acrylic acid and aliphatic alcohols such as methanol, ethanol, propanol and butanol (preferably linear or branched aliphatic alcohols having 1 to 12 carbon atoms (more preferably 1 to 6 carbon atoms)). The aliphatic alcohols may optionally be substituted with an aliphatic amino group such as a dimethylamino group, a trimethylammonium group (a primary to tertiary amino group or a quaternary ammonium group; the aliphatic group contained in the aliphatic amino group is preferably a linear or branched aliphatic group having 1 to 12 carbon atoms (more preferably, 1 to 6 carbon atoms)), a hydroxy group or a phosphorylcholine group.

Examples of monomers that derive structural unit X include acrylic acid, methacrylic acid, methyl acrylate, methyl methacrylate, ethyl acrylate, ethyl methacrylate, butyl acrylate, butyl methacrylate, octyl acrylate, octyl methacrylate, 2-ethylhexyl acrylate, 2-ethylhexyl methacrylate, dodecyl acrylate, dodecyl methacrylate, 2-hydroxyethyl acrylate, 2-hydroxyethyl methacrylate, dimethylaminoethyl acrylate, dimethylaminoethyl methacrylate, trimethylammoniumethyl acrylate chloride, trimethylammoniumethyl methacrylate chloride, 2-acryloyloxyethyl phosphorylcholine, and 2-methacryloyloxyethyl phosphorylcholine. These may be used singly or in combinations of two or more thereof.

In the present specification, the (meth)acrylic acid-based polymers may be either a homopolymer composed of a single species of monomer or a copolymer composed of a plurality of different species of monomers.

The (meth)acrylic acid-based polymers preferably contain at least one structural unit selected from the group consisting of structural units derived from (meth) acrylic acid esters and structural units derived from (meth)acrylic acid, which have one or more substituents selected from the group consisting of a primary to tertiary amino group, a quaternary ammonium group, a hydroxy group, and a phosphorylcholine group (hereinafter, also referred to as structural unit Y). Structural unit Y is preferably a structural unit derived from di-C1-C12 alkylamino C1-C12 alkyl (meth)acrylate, a structural unit derived from tri-C1-C12 alkylammonium C1-C12 alkyl (meth)acrylate, and a structural unit derived from (meth)acrylic acid. Among polymers containing structural unit Y, polymers containing structural unit Y and a structural unit derived from C1-C12 alkyl (meth)acrylate (hereinafter, also referred to as structural unit Z) are preferred (the polymers are also referred to as polymers P hereinafter).

In this specification, the (meth)acrylic acid-based polymers may contain a structural unit derived from a monomer (vinyl acetate, vinylpyrrolidone, 2-methyl-5-vinylpyridine, etc.) having a reactive unsaturated group copolymerizable with the structural unit X, in addition to the structural unit X. In this case, the content ratio of the structural unit X to the total amount of the structural units constituting the (meth) acrylic acid-based polymers is not particularly limited.

In the present specification, the average molecular weight of the (meth)acrylic acid-based polymers is not particularly limited, but preferably from 1000 to 1000000, more preferably from 10000 to 500000, further preferably from 50000 to 300000, and still further preferably from 100000 to 200000.

The average molecular weight refers to a value converted into sodium polyacrylate, and can be measured by a size exclusion chromatography method using a calibration curve prepared using a sodium polyacrylate standard manufactured by Polymer Laboratories Ltd..

The (meth)acrylic acid-based polymers include, for example, acrylic acid/octyl acrylate copolymer, acrylic acid ester/vinyl acetate copolymer, 2-ethylhexyl acrylate/vinylpyrrolidone copolymer, 2-ethylhexyl acrylate/2-ethylhexyl methacrylate/dodecyl methacrylate copolymer, ethyl acrylate/methyl methacrylate copolymer, acrylic acid silk fibroin copolymer resin, methyl acrylate/2-ethylhexyl acrylate copolymer resin, aminoalkyl methacrylate copolymer E, ammonioalkyl methacrylate copolymer, carboxyvinyl polymer, dimethylaminoethyl methacrylate/methyl methacrylate copolymer, sodium polyacrylate, partially neutralized polyacrylate, methacrylic acid/n-butyl acrylate copolymer, methacrylic acid copolymer L, methacrylic acid copolymer LD (including dried methacrylic acid copolymer LD), methacrylic acid copolymer S, methyl acrylate/ methacrylic acid copolymer, methyl acrylate/methacrylic acid/methyl methacrylate copolymer, 2-methyl-5-vinylpyridine methyl acrylate/methacrylic acid copolymer, methyl methacrylate/methacrylic acid copolymer, and the like.

The (meth)acrylic acid-based polymers are preferably polymers derived from one or more monomers selected from the group consisting of acrylic acid, methacrylic acid, methyl methacrylate, ethyl acrylate, butyl methacrylate, dimethylaminoethyl methacrylate, and trimethylammoniumethyl methacrylate chloride, from the standpoint of improving the content uniformity, and are particularly preferably at least one selected from the group consisting of ethyl acrylate/methyl methacrylate copolymer, aminoalkyl methacrylate copolymer E, ammonioalkyl methacrylate copolymer, carboxyvinyl polymer, methacrylic acid copolymer S, methacrylic acid copolymer L, and methacrylic acid copolymer LD (including dried methacrylic acid copolymer LD). As the methacrylic acid copolymer LD, a dried methacrylic acid copolymer LD is preferable.

All of these (meth) acrylic acid-based polymers are known components, and may be produced by a known method, or commercial products may be used. Examples of such commercially available products include EUDRAGIT E100, EUDRAGIT EPO, EUDRAGIT L100, EUDRAGIT L30D-55, EUDRAGIT L100-55, EUDRAGIT S100, EUDRAGIT RL100, EUDRAGIT RLPO, EUDRAGIT RL30D, EUDRAGIT RS100, EUDRAGIT RSPO, EUDRAGIT RS30D, EUDRAGIT NE30D, EUDRAGIT FS30D (from Evonick Röhm GmbH), POLYQUID PA-30, POLYQUID PA-30L, POLYQUID PA-30S, POLYQUID PA-100, POLYQUID LA-100, POLYQUID SA-100, POLYQUID EA-100, POLYQUID EM-30 (from Sanyo Chemical Industries, Ltd.), Kollicoat MAE30DP, Kollicoat MAE100-55, Kollicoat Smart Seal 30 D, Kollicoat IR, Kollicoat EMM30D (from BASF Japan Ltd.), HIVISWAKO 103, HIVISWAKO 104, HIVISWAKO 105 (from FUJIFILM Wako Pure Chemical Corporation), Carbopol971PNF, Carbopol974PNF, Carbopo171GNF (from CBC), etc.

The content of the (meth)acrylic acid-based polymers in the pharmaceutical composition is not particularly limited, and can be determined in appropriate consideration of the type of preparation, the sex, age and symptoms of a patient in need of the composition, and the like, but from the viewpoint of improvement of content uniformity, the total amount of (meth)acrylic acid-based polymers with respect to the total mass of the pharmaceutical composition is preferably from 0. 001 to 30 mass%, more preferably from 0.01 to 20 mass%, still more preferably from 0.1 to 10 mass%, and particularly preferably from 0.5 to 5 mass%.

The mass ratio between the content of pemafibrate, a salt thereof or a solvate thereof and the content of the (meth)acrylic acid-based polymers in the pharmaceutical composition is not particularly limited, and from the viewpoint of improvement of content uniformity, the total content of the (meth) acrylic acid-based polymers with respect to 1 part by mass of a free form of pemafibrate is preferably 0.001 to 500 parts by mass, more preferably 0.01 to 100 parts by mass, further preferably 0.5 to 50 parts by mass, still further preferably 1 to 25 parts by mass.

Herein, the dosage form of the "pharmaceutical composition" is not particularly limited, may be a solid, semisolid or liquid preparation, and can be selected according to the use purpose of the pharmaceutical composition. Examples of the dosage form of the pharmaceutical composition include dosage forms described in The Japanese Pharmacopoeia, 17th Edition, General Rules for Preparations. Specific examples of the peroral dosage form include solid preparations such as tablets (e.g. normal tablets, orally disintegrating tablets, chewable tablets, effervescent tablets, dispersion tablets, soluble tablets and controlled-release tablets), capsules, granules (e.g. effervescent granules), powders and pills; semisolid preparations such as peroral jellies; liquid preparations such as peroral liquids (e.g. elixirs, suspensions, emulsions and lemonades). Examples of the parenteral dosage form include injections, inhalations, eye drops, ear drops, nasal drops, suppositories, solid external preparations, liquid external preparations, sprays, ointments, creams, gels and patches.

The pharmaceutical composition is preferably a solid preparation from the viewpoint of ease of administration and ease of production. In particular, when the pharmaceutical composition is a solid preparation, production is very easy, but since in general, a solid preparation is produced basically with solid components used in a large amount at normal temperature (any temperature in the range of 15 to 25°C), components are apt to be unevenly mixed and dispersed, so that deterioration in content uniformity is apt to be particularly problematic. On the other hand, the present invention exhibits the following excellent advantage: even a solid preparation has good content uniformity.

The solid preparation is preferably a peroral solid preparation, more preferably a tablet, a capsule, a granule, a dispersion or a pill, particularly preferably a tablet. In addition, the solid preparation is preferably a solid preparation containing a mixture comprising component (A) and component (B).

In addition to the above-described components, pharmaceutically acceptable carriers (additives for pharmaceutical preparation) may be added to the pharmaceutical composition of the present invention depending on its dosage form. Examples of the additives for pharmaceutical preparation include, but are not limited to, diluents, disintegrants, binders, lubricants, plasticizers, film formers, antioxidants, flavors and sweetening agents. As specific examples of these additives for pharmaceutical preparation, those described in Japanese Pharmaceutical Excipients Directory 2016 (issued by Yakuji Nippo, Limited), Handbook of Pharmaceutical Excipients, Seventh Edition (issued by Pharmaceutical Press), etc. may be used.

Specific examples of the diluents include inorganic diluents such as anhydrous sodium sulfate, anhydrous dibasic calcium phosphate, sodium chloride, calcium sulfate, calcium monohydrogen phosphate, dibasic calcium phosphate, dibasic sodium phosphate, monobasic potassium phosphate, monobasic calcium phosphate and monobasic sodium phosphate; and organic diluents such as fructose, caramel, agar, paraffin, crystalline cellulose, sucrose, maltose, lactose, lactose monohydrate, white soft sugar, glucose, pullulan, polyoxyethylene hydrogenated castor oil, trehalose, reduced palatinose, maltose, polyvinylacetal diethylaminoacetate and calcium citrate. These diluents may be used singly, or in combinations of two or more thereof.

The total content of the diluents is not particularly limited, and preferably 20 to 99 mass%, more preferably 30 to 97 mass%, with respect to the total mass of the pharmaceutical composition.

Specific examples of the disintegrants include gelatin, sodium hydrogen carbonate, dextrin, dehydroacetic acid and salts thereof and polyoxyethylene hydrogenated castor oil 60. These disintegrants may be used singly, or in combinations of two or more thereof.

Specific examples of the binders include dextrin, pullulan, acacia, agar, gelatin, tragacanth, sodium alginate and polyvinylacetal diethylaminoacetate. These binders may be used singly, or in combinations of two or more thereof.

Specific examples of the lubricants include calcium stearate, magnesium stearate and sodium stearyl fumarate. Theses lubricants may be used singly, or in combinations of two or more thereof.

The total content of the lubricants is not particularly limited, and preferably 0.01 to 15 mass%, more preferably 0.1 to 10 mass%, with respect to the total mass of the pharmaceutical composition.

Specific examples of the plasticizers include sesame oil, castor oil and polysorbate 80 (polyoxyethylene (20) sorbitan oleate ester). These plasticizers may be used singly, or in combinations of two or more thereof.

Specific examples of the film formers include alginic acid or salts thereof such as sodium alginate, carrageenan, xanthan gum and pullulan. These film formers may be used singly, or in combinations of two or more thereof.

Specific examples of the antioxidants include ascorbic acid, sodium hydrogen sulfite, sodium sulfite, sodium edetate, erythorbic acid, tocopherol acetate, dibutylhydroxytoluene, natural vitamin E, tocopherol and butylhydroxyanisole. These antioxidants may be used singly, or in combinations of two or more thereof.

Specific examples of the flavors include terpenes such as limonene, pinene, camphene, cymene, cineole, citronellol, geraniol, nerol, linalool, menthol, terpineol, rhodinol, borneol, isoborneol, menthone, camphor, eugenol and cinnzeylanol; terpene-containing essential oils such as bitter orange oil, orange oil, peppermint oil, camphor white oil, eucalyptus oil, turpentine oil, lemon oil, ginger oil, clove oil, cinnamon oil, lavender oil, fennel oil, chamomile oil, fermented soybean oil and spearmint oil; and acidulants such as ascorbic acid, tartaric acid, citric acid, malic acid and salts thereof. These flavors may be used singly, or in combinations of two or more thereof.

Examples of the sweetening agents include aspartame, stevia, sucralose, glycyrrhizic acid, thaumatin, acesulfame potassium, saccharin and saccharin sodium, and these sweetening agents may be used singly, or in combinations of two or more thereof.

The pharmaceutical composition of the present invention can be produced through a known method depending on its dosage form.

For example, the pharmaceutical composition, when it is a solid preparation, can be produced through appropriate combination of unit operations such as grinding, mixing, granulation, drying, grain size adjustment, classification, filling, pelletizing and coating. However, the method for producing the same preferably involves a step of mixing component (A) and component (A).

More specifically, for example, when the dosage form of the pharmaceutical composition is a granular preparation such as a granule, a powder or a pill, component (A) and component (B) are mixed with additives for pharmaceutical preparation such as diluents, binders, disintegrants and lubricants in accordance with needs, the mixture is then granulated through a known granulation method such as extrusion granulation, tumbling granulation, agitation granulation, fluidized bed granulation, spray granulation, melt granulation or crushing granulation to obtain a granulated product, and the granulated product is subjected to classification, grain size adjustment and the like in accordance with needs, whereby the pharmaceutical composition can be produced. The obtained granulated product can be coated through a known method with a coating agent etc.

When the dosage form of the pharmaceutical composition is a tablet, component (A) and component (B) are mixed with additives for pharmaceutical preparation such as diluents, binders, disintegrants and lubricants in accordance with needs to obtain a mixture, and the mixture is directly compressed (pelletized) (through a direct powder compression method), or compressed (pelletized) (through a semidry grain compression method, dry granule compression method, wet grain compression method or the like) after the above-described granulated product is subjected to classification, grain size adjustment and the like in accordance with needs, whereby the pharmaceutical composition can be produced. The obtained compressed product (tablet) can be coated through a known method with a coating agent etc.

When the dosage form of the pharmaceutical composition is a capsule, the granulated product or compressed product may be capsulated.

The disease to which the pharmaceutical composition of the present invention is applied is not limited, and the pharmaceutical composition can be widely used for prevention or treatment of diseases against which administration of pemafibrate is known or expected to be effective.

For example, pemafibrate, a salt thereof or a solvate thereof has excellent PPAR-α agonist activity, and exhibits plasma triglyceride concentration reducing action, HDL cholesterol increasing action, etc. Therefore, the pharmaceutical composition of the present invention can be used preferably as an agent for prevention and/or treatment of dyslipidemia (hyperlipidemia, more specifically, for example primary hyperlipidemia and secondary hyperlipidemia), further preferably as an agent for prevention and/or treatment of hypertriglyceridemia, etc.

In addition, pemafibrate, a salt thereof or a solvate thereof is useful for prevention or treatment of NAFLD (non-alcoholic fatty liver disease). Therefore, the pharmaceutical composition of the present invention can also be used as an agent for prevention and/or treatment of NAFLD (more preferably NASH (non-alcoholic steatohepatitis)), etc.

Further, pemafibrate, a salt thereof or a solvate thereof may be used as an agent for treatment of primary biliary cirrhosis, etc.

The administration route of the pharmaceutical composition is not particularly limited, and can be determined in appropriate consideration of the target disease, the type of preparation, the sex, age, symptoms of a patient in need of the composition, and the like, but peroral administration is preferable from the viewpoint of ease of administration. The daily dose of the pharmaceutical composition can be taken as a single dose, or can be divided into 2 to 4 daily administrations (preferably taken as a single dose), and taken before each meal, between meals, after each meal, before bedtime, or the like.

For example, the following aspects are disclosed herein and should not be construed as limiting the present invention.
[1-1] A pharmaceutical composition comprising the following components (A) and (B):
   (A) pemafibrate, a salt thereof or a solvate thereof; and
   (B) one or more selected from the group consisting of the following components (B-1) to (B-7):
      (B-1) a cellulose ether species;
      (B-2) a starch species;
      (B-3) a povidone species;
      (B-4) a silicic acid compound;
      (B-5) a polyhydric alcohol;
      (B-6) an alkyl sulfate ester; and
      (B-7) a (meth)acrylic acid-based polymer.
[1-2] The pharmaceutical composition according to [1-1], wherein the component (B-1) is one or more selected from the group consisting of an alkylcellulose, a hydroxyalkylcellulose, an alkyl(hydroxyalkyl)cellulose, a carboxyalkylcellulose, a cross-linked polymer of a carboxyalkylcellulose and a salt thereof.
[1-3] The pharmaceutical composition according to [1-1], wherein the component (B-1) is one or more selected from the group consisting of a C1-C6 alkylcellulose, a hydroxy C1-C6 alkylcellulose, a C1-C6 alkyl(hydroxy C1-C6 alkyl)cellulose, a carboxy C1-C6 alkylcellulose, a cross-linked polymer of a carboxy C1-C6 alkylcellulose and a salt thereof.
[1-4] The pharmaceutical composition according to [1-1], wherein the component (B-1) is one or more selected from the group consisting of methylcellulose, ethylcellulose, hydroxypropylcellulose, hypromellose, carmellose, carmellose potassium, carmellose calcium, carmellose sodium and croscarmellose sodium.
[1-5] The pharmaceutical composition according to any one of [1-1] to [1-4], wherein the component (B-2) is one or more selected from the group consisting of starch, a hydroxyalkyl ether of starch, a carboxyalkyl ether of starch and a salt thereof.
[1-6] The pharmaceutical composition according to any one of [1-1] to [1-4], wherein the component (B-2) is one or more selected from the group consisting of starch, a hydroxy C1-C6 alkyl ether of starch, a carboxy C1-C6 alkyl ether and a salt thereof.
[1-7] The pharmaceutical composition according to any one of [1-1] to [1-4], wherein the component (B-2) is one or more selected from the group consisting of starch, hydroxypropyl starch, carboxymethyl starch and a salt thereof.
[1-8] The pharmaceutical composition according to any one of [1-1] to [1-7], wherein the component (B-3) is one or more selected from the group consisting of povidone and crospovidone.
[1-9] The pharmaceutical composition according to any one of [1-1] to [1-7], wherein the component (B-3) is crospovidone.
[1-10] The pharmaceutical composition according to any one of [1-1] to [1-9], wherein the component (B-4) is one or more selected from the group consisting of a hydrous silicic acid compound, a salt of a hydrous silicic acid compound, an anhydrous silicic acid and a salt of an anhydrous silicic acid.
[1-11] The pharmaceutical composition according to any one of [1-1] to [1-9], wherein the component (B-4) is one or more selected from the group consisting of hydrous magnesium silicate, hydrated silicon dioxide and light anhydrous silicic acid.
[1-12] The pharmaceutical composition according to any one of [1-1] to [1-9], wherein the component (B-4) is one or more selected from the group consisting of hydrous magnesium silicate and hydrated silicon dioxide.
[1-13] The pharmaceutical composition according to any one of [1-1] to [1-12], wherein the component (B-5) is macrogol.
[1-14] The pharmaceutical composition according to any one of [1-1] to [1-12], wherein the component (B-5) is one or more selected from the group consisting of macrogol 100, macrogol 200, macrogol 300, macrogol 400, macrogol 600, macrogol 1000, macrogol 1500, macrogol 1540, macrogol 4000, macrogol 6000, polyethylene glycol 8000, macrogol 20000 and macrogol 35000.
[1-15] The pharmaceutical composition according to any one of [1-1] to [1-12], wherein the component (B-5) is macrogol having an average molecular weight of 100 to 10,000.
[1-16] The pharmaceutical composition according to any one of [1-1] to [1-12], wherein the component (B-5) is macrogol 6000.
[1-17] The pharmaceutical composition according to any one of [1-1] to [1-12], wherein the component (B-5) is one or more selected from the group consisting of erythritol, xylitol, mannitol, sorbitol, maltitol and lactitol.
[1-18] The pharmaceutical composition according to any one of [1-1] to [1-12], wherein the component (B-5) is one or more selected from the group consisting of mannitol and sorbitol.
[1-19] The pharmaceutical composition according to any one of [1-1] to [1-12], wherein the component (B-5) is mannitol.
[1-20] The pharmaceutical composition according to any one of [1-1] to [1-19], wherein the component (B-6) is one or more selected from the group consisting of a lauryl sulfate ester salt, a tetradecyl sulfate ester salt, a hexadecyl sulfate ester salt and an octadecyl sulfate ester salt.
[1-21] The pharmaceutical composition according to any one of [1-1] to [1-19], wherein the component (B-6) is a lauryl sulfate ester salt.
[1-22] The pharmaceutical composition according to any one of [1-1] to [1-19], wherein the component (B-6) is sodium lauryl sulfate.
[1-23] The pharmaceutical composition according to any one of [1-1] to [1-22], wherein the component (B-7) is a polymer derived from at least one monomer selected from the group consisting of acrylic acid, methacrylic acid, methyl methacrylate, ethyl acrylate, butyl methacrylate, dimethylaminoethyl methacrylate and trimethylammoniumethyl methacrylate chloride.
[1-24] The pharmaceutical composition according to any one of [1-1] to [1-22], wherein the component (B-7) is one or more selected from the group consisting of ethyl acrylate/methyl methacrylate copolymer, aminoalkyl methacrylate copolymer E, ammonioalkyl methacrylate copolymer, carboxyvinyl polymer, methacrylic acid copolymer S, methacrylic acid copolymer L and methacrylic acid copolymer LD.
[1-25] The pharmaceutical composition according to any one of [1-1] to [1-22], wherein the component (B-7) is one or more selected from the group consisting of aminoalkyl methacrylate copolymer E, ammonioalkyl methacrylate copolymer, methacrylic acid copolymer S, methacrylic acid copolymer L and methacrylic acid copolymer LD.
[1-26] The pharmaceutical composition according to any one of [1-1] to [1-25], wherein the pharmaceutical composition is an agent for prevention and/or treatment of a disease selected from dyslipidemia (hyperlipidemia, more specifically, for example primary hyperlipidemia and secondary hyperlipidemia), NAFLD (more preferably NASH (non-alcoholic steatohepatitis)) and primary biliary cirrhosis.
[1-27] The pharmaceutical composition according to any one of [1-1] to [1-26], wherein the pharmaceutical composition is a solid preparation.
[1-28] The pharmaceutical composition according to any one of [1-1] to [1-27], wherein a dosage form thereof is a tablet, a capsule, a granule, a powder or a pill.
[2-1] A method for improving content uniformity of pemafibrate, a salt thereof or a solvate thereof in a pharmaceutical composition, the method comprising the step of incorporating one or more selected from the group consisting of the following components (B-1) to (B-7):
   (B-1) a cellulose ether species;
   (B-2) a starch species;
   (B-3) a povidone species;
   (B-4) a silicic acid compound;
   (B-5) a polyhydric alcohol;
   (B-6) an alkyl sulfate ester; and
   (B-7) a (meth)acrylic acid-based polymer in a pharmaceutical composition comprising (A) pemafibrate, a salt thereof or a solvate thereof.
[2-2] The method according to [2-1], wherein the component (B-1) is one or more selected from the group consisting of an alkylcellulose, a hydroxyalkylcellulose, an alkyl(hydroxyalkyl)cellulose, a carboxyalkylcellulose, a cross-linked polymer of a carboxyalkylcellulose and a salt thereof.
[2-3] The method according to [2-1], wherein the component (B-1) is one or more selected from the group consisting of a C1-C6 alkylcellulose, a hydroxy C1-C6 alkylcellulose, a C1-C6 alkyl(hydroxy C1-C6 alkyl)cellulose, a carboxy C1-C6 alkylcellulose, a cross-linked polymer of a carboxy C1-C6 alkylcellulose and a salt thereof.
[2-4] The method according to [2-1], wherein the component (B-1) is one or more selected from the group consisting of methylcellulose, ethylcellulose, hydroxypropylcellulose, hypromellose, carmellose, carmellose potassium, carmellose calcium, carmellose sodium and croscarmellose sodium.
[2-5] The method according to any one of [2-1] to [2-4], wherein the component (B-2) is one or more selected from the group consisting of starch, a hydroxyalkyl ether of starch, a carboxyalkyl ether of starch and a salt thereof.
[2-6] The method according to any one of [2-1] to [2-4], wherein the component (B-2) is one or more selected from the group consisting of starch, a hydroxy C1-C6 alkyl ether of starch, a carboxy C1-C6 alkyl ether and a salt thereof.
[2-7] The method according to any one of [2-1] to [2-4], wherein the component (B-2) is one or more selected from the group consisting of starch, hydroxypropyl starch, carboxymethyl starch and a salt thereof.
[2-8] The method according to any one of [2-1] to [2-7], wherein the component (B-3) is one or more selected from the group consisting of povidone and crospovidone.
[2-9] The pharmaceutical composition according to any one of [2-1] to [2-7], wherein the component (B-3) is crospovidone.
[2-10] The method according to any one of [2-1] to [2-9], wherein the component (B-4) is one or more selected from the group consisting of a hydrous silicic acid compound, a salt of a hydrous silicic acid compound, an anhydrous silicic acid and a salt of an anhydrous silicic acid.
[2-11] The method according to any one of [2-1] to [2-9], wherein the component (B-4) is one or more selected from the group consisting of hydrous magnesium silicate, hydrated silicon dioxide and light anhydrous silicic acid.
[2-12] The method according to any one of [2-1] to [2-9], wherein the component (B-4) is one or more selected from the group consisting of hydrous magnesium silicate and hydrated silicon dioxide.
[2-13] The method according to any one of [2-1] to [2-12], wherein the component (B-5) is macrogol.
[2-14] The method according to any one of [2-1] to [2-12], wherein the component (B-5) is one or more selected from the group consisting of macrogol 100, macrogol 200, macrogol 300, macrogol 400, macrogol 600, macrogol 1000, macrogol 1500, macrogol 1540, macrogol 4000, macrogol 6000, polyethylene glycol 8000, macrogol 20000 and macrogol 35000.
[2-15] The method according to any one of [2-1] to [2-12], wherein the component (B-5) is macrogol having an average molecular weight of 100 to 10,000.
[2-16] The method according to any one of [2-1] to [2-12], wherein the component (B-5) is macrogol 6000.
[2-17] The method according to any one of [2-1] to [2-12], wherein the component (B-5) is one or more selected from the group consisting of erythritol, xylitol, mannitol, sorbitol, maltitol and lactitol.
[2-18] The method according to any one of [2-1] to [2-12], wherein the component (B-5) is one or more selected from the group consisting of mannitol and sorbitol.
[2-19] The method according to any one of [2-1] to [2-12], wherein the component (B-5) is mannitol.
[2-20] The method according to any one of [2-1] to [2-19], wherein the component (B-6) is one or more selected from the group consisting of a lauryl sulfate ester salt, a tetradecyl sulfate ester salt, a hexadecyl sulfate ester salt and an octadecyl sulfate ester salt.
[2-21] The method according to any one of [2-1] to [2-19], wherein the component (B-6) is a lauryl sulfate ester salt.
[2-22] The method according to any one of [2-1] to [2-19], wherein the component (B-6) is sodium lauryl sulfate.
[2-23] The method according to any one of [2-1] to [2-22], wherein the component (B-7) is a polymer derived from at least one monomer selected from the group consisting of acrylic acid, methacrylic acid, methyl methacrylate, ethyl acrylate, butyl methacrylate, dimethylaminoethyl methacrylate and trimethylammoniumethyl methacrylate chloride.
[2-24] The method according to any one of [2-1] to [2-22], wherein the component (B-7) is one or more selected from the group consisting of ethyl acrylate/methyl methacrylate copolymer, aminoalkyl methacrylate copolymer E, ammonioalkyl methacrylate copolymer, carboxyvinyl polymer, methacrylic acid copolymer S, methacrylic acid copolymer L and methacrylic acid copolymer LD.
[2-25] The method according to any one of [2-1] to [2-22], wherein the component (B-7) is one or more selected from the group consisting of aminoalkyl methacrylate copolymer E, ammonioalkyl methacrylate copolymer, methacrylic acid copolymer S, methacrylic acid copolymer L and methacrylic acid copolymer LD.
[2-26] The method according to any one of [2-1] to [2-25], wherein the pharmaceutical composition is an agent for prevention and/or treatment of a disease selected from dyslipidemia (hyperlipidemia, more specifically, for example primary hyperlipidemia and secondary hyperlipidemia), NAFLD (more preferably NASH (non-alcoholic steatohepatitis)) and primary biliary cirrhosis.
[2-27] The method according to any one of [2-1] to [2-26], wherein the pharmaceutical composition is a solid preparation.
[2-28] The method according to any one of [2-1] to [2-27], wherein a dosage form of the pharmaceutical composition is a tablet, a capsule, a granule, a powder or a pill.

### Examples

The present invention will next be described in detail by way of Examples, which should not be construed as limiting the invention thereto.

In Test Examples below, measurement was performed through HPLC using an ODS column as a column and an ultraviolet spectrophotometer as a detector.

For pemafibrate used in Test Examples below, the average particle diameters of primary particles were measured in accordance with The Japanese Pharmacopoeia, 17th Edition, Laser Diffraction Measurement of Particle Size, and the results showed that the d50 value was 100 **µ**m or less, and the d90 value was 200 **µ**m or less.

### [Test Example 1]

### Content uniformity evaluation test (1)

The following test was conducted for evaluating the uniformity of the content of pemafibrate in a pharmaceutical composition.

Tablets were produced using the components shown in Table 1 in such a manner that the amounts (mg) of the components per tablet were as shown in Table 1. Specific procedures will be described below.

### (Examples 1 to 6)

Pemafibrate and cellulose ether species were mixed for 30 seconds, lactose monohydrate and microcrystalline cellulose were added, the mixture was mixed for 30 seconds, magnesium stearate was added, and the mixture was mixed for 30 seconds. Thereafter, using a tablet press equipped with a punch having a diameter of 7 mm, the resulting mixture was compressed to obtain 1,000 tablets each having a weight of 120 mg.

### (Comparative Example 1)

Pemafibrate, lactose monohydrate and microcrystalline cellulose were mixed for 30 seconds, magnesium stearate was added, and the mixture was mixed for 30 seconds. Thereafter, using a tablet press equipped with a punch having a diameter of 7 mm, the resulting mixture was compressed to obtain 1,000 tablets each having a weight of 117.6 mg.

From the tablets obtained in Examples and Comparative Example, ten tablets were randomly picked up, and the content of pemafibrate in each tablet was measured through the following method.

One tablet was put in water to crush the tablet, and acetonitrile was then added to obtain a sample solution. The obtained sample solution was analyzed with a HPLC apparatus to measure the pemafibrate-derived peak area. By comparing the pemafibrate-derived peak area for the obtained sample solution to the peak area for a standard solution of pemafibrate with a known concentration, the pemafibrate content per tablet was measured.

From the thus-obtained measured value of the pemafibrate content per tablet, a relative standard deviation (RSD) (%) of the pemafibrate content in the tablet was calculated in accordance with The Japanese Pharmacopoeia, 17th Edition, Content Uniformity Test, and used as an index of variation (degree of uniformity) of the pemafibrate content in the tablet.

Table 1 shows the results.

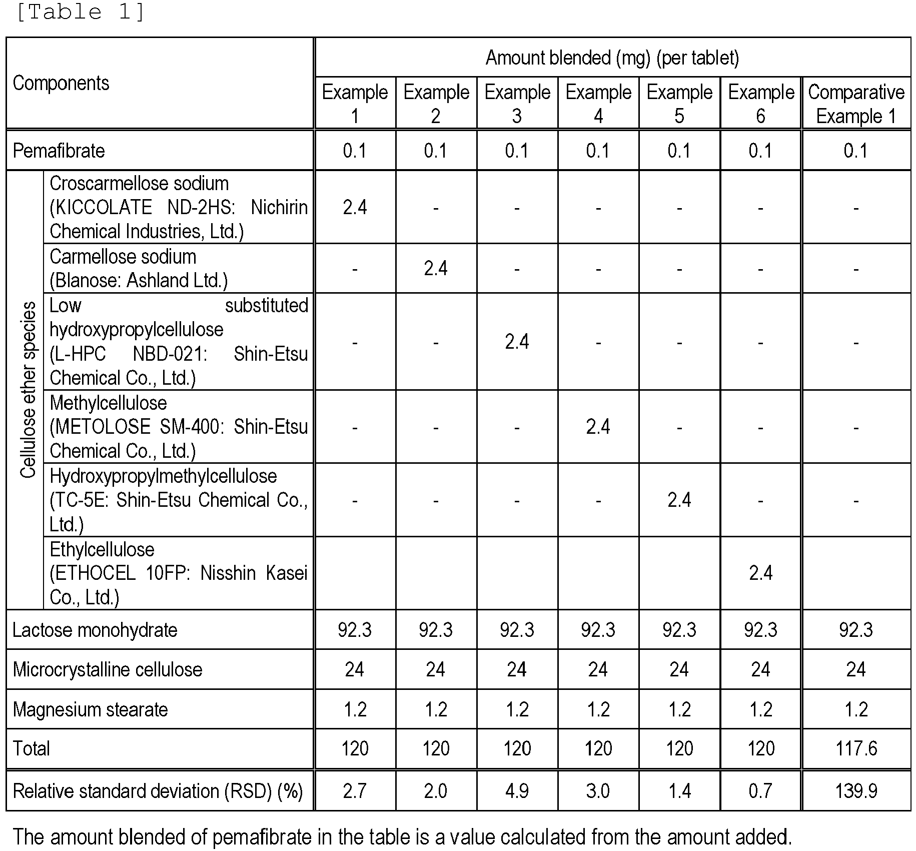

As is apparent from the results shown in Table 1, the tablets of Comparative Example 1 which did not contain cellulose ether species had a relative standard deviation of about 140% and poor uniformity of the content of pemafibrate per tablet.

In contrast, the tablets containing croscarmellose sodium, carmellose sodium, low substituted hydroxypropylcellulose, methylcellulose, hydroxypropylmethylcellulose or ethylcellulose as cellulose ether species (Examples 1 to 6) all had a small relative standard deviation and good uniformity of the content pemafibrate per tablet. The amount of cellulose ether species added was as small as 2.4 mg (2 mass% with respect to the total mass of the tablet).

The above test results reveal that the content uniformity of pemafibrate in the pharmaceutical composition is improved by incorporating cellulose ether species in a pharmaceutical composition containing pemafibrate, a salt thereof or a solvate thereof.

### [Test Example 2]

### Content uniformity evaluation test (2)

A test was conducted through the same method as in Test Example 1 except that tablets had compositions in which components and the amounts thereof were as shown in Table 2 below.

Table 2 shows the results.

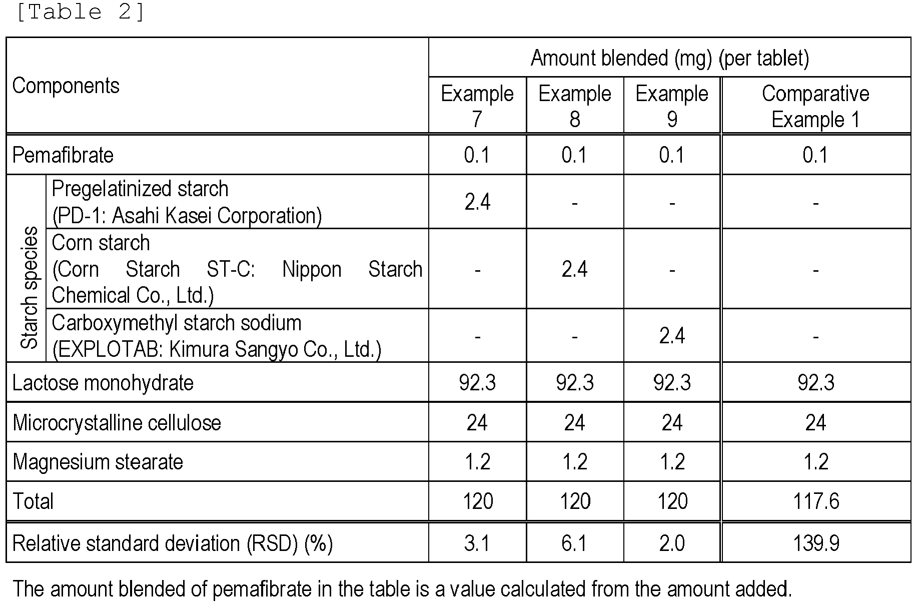

As is apparent from the results shown in Table 2, the tablets containing pregelatinized starch, corn starch or carboxymethyl starch sodium as starch species (Examples 7 to 9) all had a small relative standard deviation and good uniformity of the content pemafibrate per tablet as did the tablets of Examples 1 to 6 containing cellulose ether species in Test Example 1.

The above test results reveal that the content uniformity of pemafibrate in the pharmaceutical composition is improved by incorporating starch species in a pharmaceutical composition containing pemafibrate, a salt thereof or a solvate thereof.

### [Test Example 3]

### Content uniformity evaluation test (3)

A test was conducted through the same method as in Test Example 1 except that tablets had compositions in which components and the amounts thereof were as shown in Table 3 below.

Table 3 shows the results.

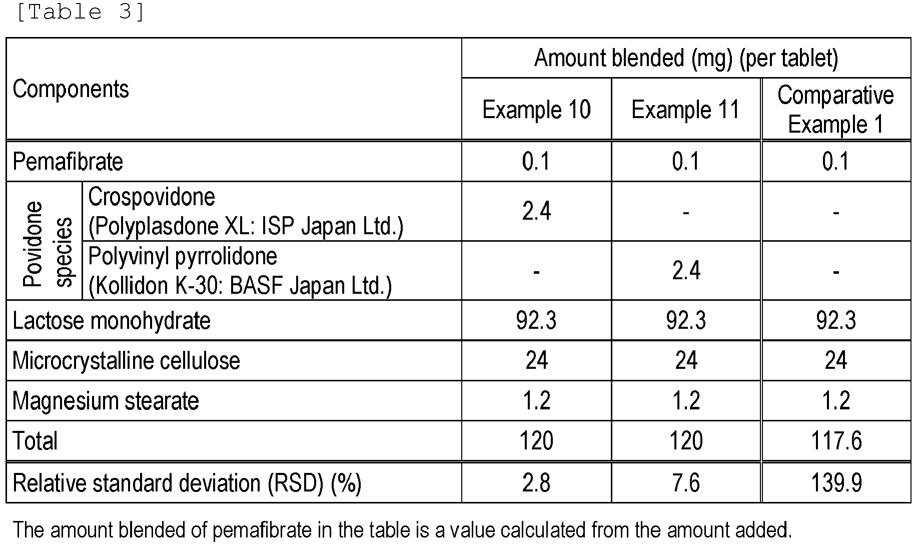

As is apparent from the results shown in Table 3, the tablets containing crospovidone or polyvinyl pyrrolidone as povidone species (Examples 10 and 11) both had a small relative standard deviation and good uniformity of the content pemafibrate per tablet as did the tablets of Examples 1 to 6 containing cellulose ether species in Test Example 1.

The above test results reveal that the content uniformity of pemafibrate in the pharmaceutical composition is improved by incorporating povidone species in a pharmaceutical composition containing pemafibrate, a salt thereof or a solvate thereof.

### [Test Example 4]

### Content uniformity evaluation test (4)

A test was conducted through the same method as in Test Example 1 except that tablets had compositions in which components and the amounts thereof were as shown in Table 4 below.

Table 4 shows the results.

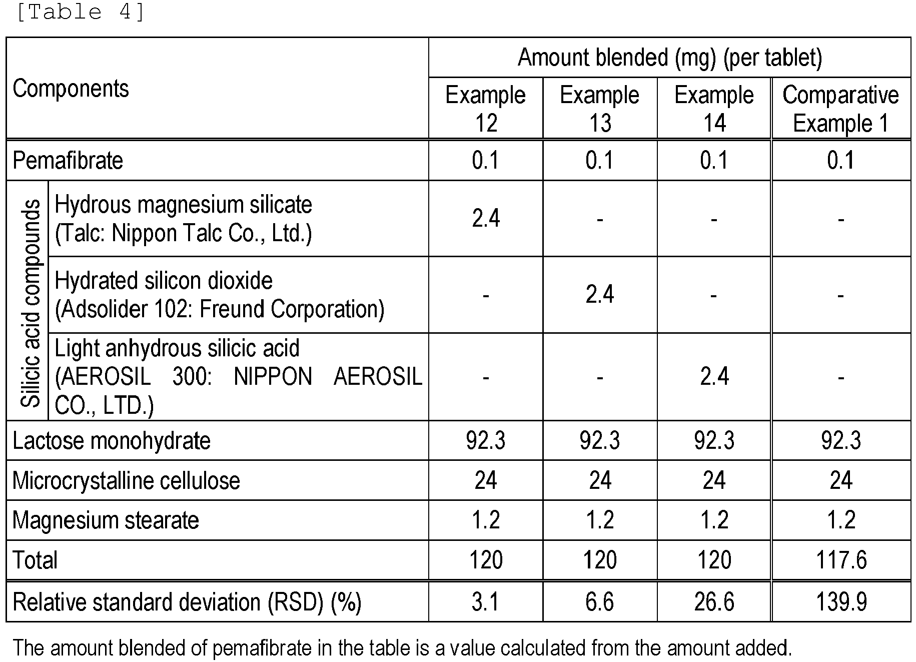

As is apparent from the results shown in Table 4, the tablets containing hydrous magnesium silicate, hydrated silicon dioxide or light anhydrous silicic acid as silicic acid compound (Examples 12 to 14) all had a small relative standard deviation and good uniformity of the content pemafibrate per tablet as did the tablets of Examples 1 to 6 containing cellulose ether species in Test Example 1.

The above test results reveal that the content uniformity of pemafibrate in the pharmaceutical composition is improved by incorporating silicic acid compound in a pharmaceutical composition containing pemafibrate, a salt thereof or a solvate thereof.

### [Test Example 5]

### Content uniformity evaluation test (5)

A test was conducted through the same method as in Test Example 1 except that tablets had compositions in which components and the amounts thereof were as shown in Table 5 below.

Table 5 shows the results.

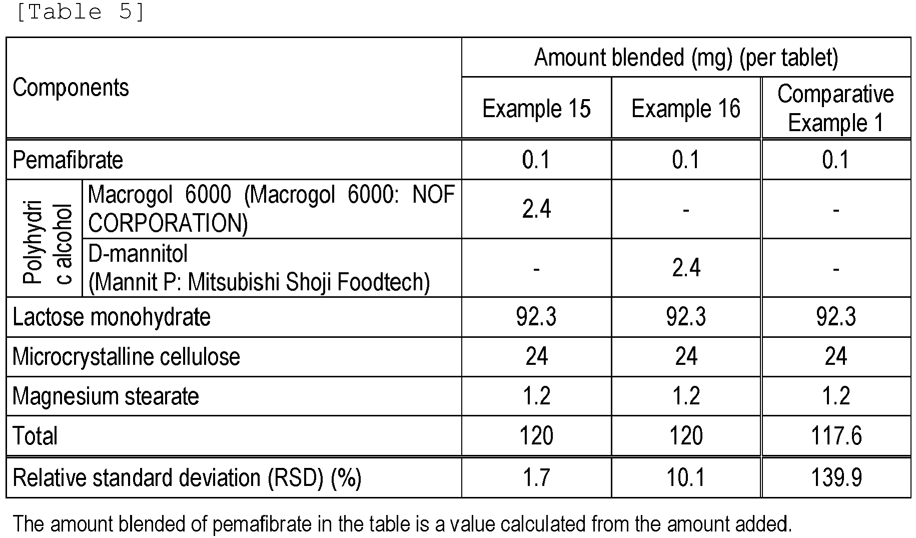

As is apparent from the results shown in Table 5, the tablets containing macrogol or mannitol as polyhydric alcohol (Examples 15 and 16) both had a small relative standard deviation and good uniformity of the content pemafibrate per tablet as did the tablets of Examples 1 to 6 containing cellulose ether species in Test Example 1.

The above test results reveal that the content uniformity of pemafibrate in the pharmaceutical composition is improved by incorporating polyhydric alcohol in a pharmaceutical composition containing pemafibrate, a salt thereof or a solvate thereof.

### [Test Example 6]

### Content uniformity evaluation test (6)

A test was conducted through the same method as in Test Example 1 except that tablets had compositions in which components and the amounts thereof were as shown in Table 6 below.

Table 6 shows the results.

**[Table 6]**

| Components | Amount blended (mg) (per tablet) | |
|---|---|---|
| | Example 17 | Comparative Example 1 |
| Pemafibrate | 0.1 | 0.1 |
| Sodium lauryl sulfate | 2.4 | - |
| (Sodium Lauryl Sulfate: Wako Pure Chemical Industries, Ltd.) | | |
| Lactose monohydrate | 92.3 | 92.3 |
| Microcrystalline cellulose | 24 | 24 |
| Magnesium stearate | 1.2 | 1.2 |
| Total | 120 | 117.6 |
| Relative standard deviation (RSD) (%) | 1.0 | 139.9 |

| | | |
|---|---|---|
| The amount blended of pemafibrate in the table is a value calculated from the amount added. | | |

As is apparent from the results shown in Table 6, the tablets of Example 17 containing sodium lauryl sulfate as alkyl sulfate ester had a small relative standard deviation and good uniformity of the content pemafibrate per tablet as did the tablets of Examples 1 to 6 containing cellulose ether species in Test Example 1.

The above test results reveal that the content uniformity of pemafibrate in the pharmaceutical composition is improved by incorporating alkyl sulfate ester in a pharmaceutical composition containing pemafibrate, a salt thereof or a solvate thereof.

### [Test Example 7]

### Content uniformity evaluation test (7)

A test was conducted through the same method as in Test Example 1 except that tablets had compositions in which components and the amounts thereof were as shown in Table 7 below.

Table 7 shows the results.

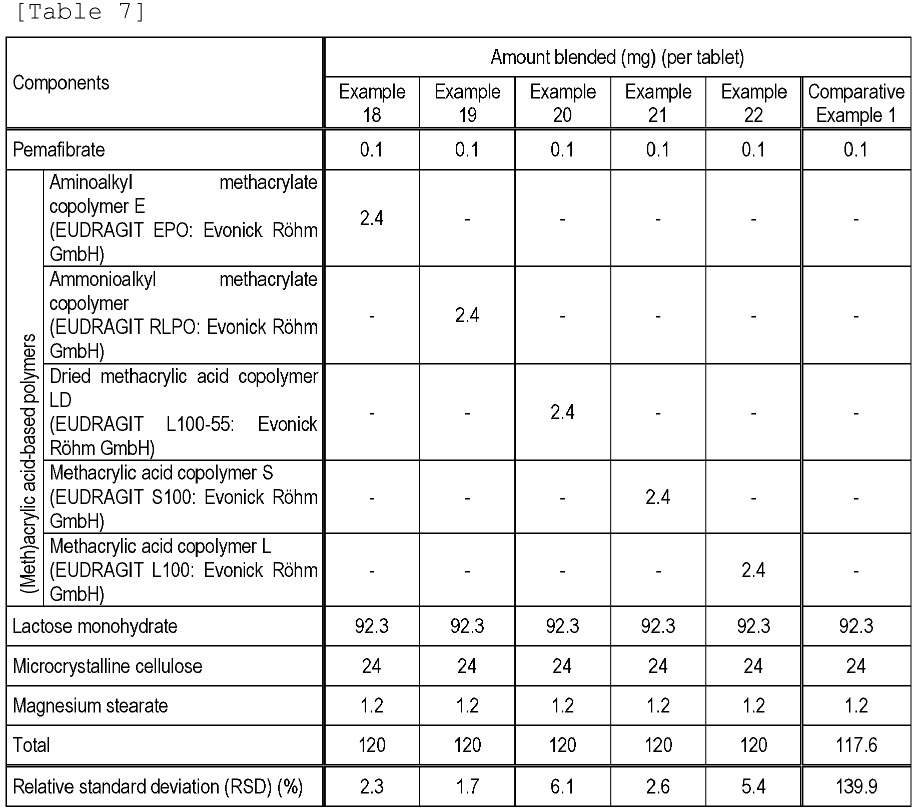

As is apparent from the results shown in Table 7, the tablets containing ammonioalkyl methacrylate copolymer, aminoalkyl methacrylate copolymer E, dried methacrylic acid copolymer LD, methacrylic acid copolymer S or methacrylic acid copolymer L as (meth)acrylic acid-based polymers (Examples 18 to 22) all had a small relative standard deviation and good uniformity of the content pemafibrate per tablet as did the tablets of Examples 1 to 6 containing cellulose ether species in Test Example 1.

The above test results reveal that the content uniformity of pemafibrate in the pharmaceutical composition is improved by incorporating (meth)acrylic acid-based polymers in a pharmaceutical composition containing pemafibrate, a salt thereof or a solvate thereof.

### [Production Examples 1 to 6]

Tablets containing the components in the amounts (mg) thereof per tablet shown in Tables 8 and 9 are conventionally produced through a wet grain compression method.

**[Table 8]**

| Components | Amount blended (mg) (per tablet) | | |
|---|---|---|---|
| | Production Example 1 | Production Example 2 | Production Example 3 |
| Pemafibrate | 0.1 | 0.4 | 0.1 |
| Lactose monohydrate | q.s. | q.s. | q.s. |
| Magnesium stearate | 1.2 | 1.2 | 1.2 |
| Carmellose sodium | 3 | | 6 |
| Croscarmellose sodium | 1 | | |
| Low substituted hydroxypropylcellulose | | 2 | 4 |
| Crospovidone | | 3 | |
| Carmellose calcium | 2 | | |
| Povidone K25 | | 1 | |
| Ethylcellulose | 0.3 | 5 | |
| Hydroxyethylmethylcellulose | | 1 | |
| Hypromellose acetate succinate | | | 4 |
| Hypromellose phthalate | | | 6 |
| Hydroxyethylcellulose | 1 | | 2 |
| Hydroxypropylcellulose | 1 | | |
| Hypromellose | | 1 | |
| Methylcellulose | 1 | | |
| Polyvinyl alcohol (partially saponified) | | | 1 |
| Magnesium aluminosilicate | | | 3 |
| Aluminum magnesium silicate | | | 2 |
| Total | 100 mg | 100 mg | 100 mg |

| | | | |
|---|---|---|---|
| The amount blended of pemafibrate in the table is a value calculated from the amount added. | | | |

**[Table 9]**

| Components | Amount blended (mg) (per tablet) | | |
|---|---|---|---|
| | Production Example 4 | Production Example 5 | Production Example 6 |
| Pemafibrate | 0.2 | 0.4 | 0.2 |
| Lactose monohydrate | q.s. | q.s. | q.s. |
| Magnesium stearate | 1.2 | 1.2 | 1.2 |
| Carmellose sodium | | | 1 |
| Croscarmellose sodium | 2 | | 1 |
| Carmellose | | 5 | |
| Carmellose potassium | | | 3 |
| Povidone K90 | 4 | | |
| Copolyvidone | | 3 | |
| Hydroxyethylmethylcellulose | | 9 | |
| Hypromellose acetate succinate | | | 2 |
| Carboxymethylethylcellulose | 5 | | 2 |
| Hydroxypropylcellulose | 1 | | |
| Hypromellose | | 0.5 | |
| Methylcellulose | 25 | 0.03 | 3 |
| Potato starch | | 15 | |
| Macrogol 400 | | | 2 |
| Macrogol 4000 | | | 2 |
| Macrogol 6000 | | 6 | |
| Polyvinyl alcohol (fully saponified) | 1 | | |
| Calcium silicate | 1 | | |
| Hydrous magnesium silicate | | 5 | |
| Magnesium aluminometasilicate | | 1 | |
| Meglumine | | | 0.5 |
| Bentonite | | | 6 |
| Total | 100 mg | 100 mg | 100 mg |

| | | | |
|---|---|---|---|
| The amount blended of pemafibrate in the table is a value calculated from the amount added. | | | |

### [Production Examples 7 to 12]

Tablets containing the components in the amounts (mg) thereof per tablet shown in Tables 10 and 11 are conventionally produced through a direct powder compression method.

**[Table 10]**

| Components | Amount blended (mg) (per tablet) | | |
|---|---|---|---|
| | Production Example 7 | Production Example 8 | Production Example 9 |
| Pemafibrate | 0.1 | 0.4 | 0.1 |
| Lactose monohydrate | q.s. | q.s. | q.s. |
| Magnesium stearate | 1.2 | 1.2 | 1.2 |
| Methylcellulose | | 7 | 2 |
| Ethylcellulose | 13 | | 2 |
| Pregelatinized starch | 3 | | |
| Wheat starch | 15 | | |
| Rice starch | | 10 | |
| Corn starch | | 20 | |
| Partially pregelatinized starch | | | 3 |
| Wheat flour | | | 10 |
| Hydrated silicon dioxide | | 1 | |
| Hydrous amorphous silicon oxide | | | 0.5 |
| Kaolin | | | 0.4 |
| Talc | 5 | | |
| Sodium lauryl sulfate | 0.3 | | |
| Erythritol | 20 | | |
| Xylitol | | 30 | |
| D-mannitol | | | 40 |
| Propylene glycol | | | 1 |
| Total | 100 mg | 100 mg | 100 mg |

| | | | |
|---|---|---|---|
| The amount blended of pemafibrate in the table is a value calculated from the amount added. | | | |

**[Table 11]**

| Components | Amount blended (mg) (per tablet) | | |
|---|---|---|---|
| | Production Example 10 | Production Example 11 | Production Example 12 |
| Pemafibrate | 0.2 | 0.4 | 0.2 |
| Lactose monohydrate | q.s. | q.s. | q.s. |
| Magnesium stearate | 1.2 | 1.2 | 1.2 |
| Methylcellulose | | 6 | |
| Ethylcellulose | 0.7 | | 4 |
| Pregelatinized starch | | | 2 |
| Partially pregelatinized starch | | | 6 |
| Rice flour | 10 | | |
| Semi-digested starch | 5 | | |
| Hydroxypropyl starch | | 8 | 1 |
| Carboxymethyl starch sodium (sodium carboxymethyl starch) | | 3 | |
| Light anhydrous silicic acid | 2 | | |
| Heavy anhydrous silicic acid | | 0.3 | |
| Silica | | | 0.1 |
| Natural aluminum silicate | | | 0.8 |
| Synthetic aluminum silicate | | 0.7 | |
| Diatomaceous earth | 0.2 | | |
| D-sorbitol | 50 | | |
| Maltitol | | 60 | |
| Lactitol | | | 70 |
| Polyoxyethylene (105) polyoxypropylene (5) glycol | | 0.5 | |
| Polyoxyethylene (160) polyoxypropylene (30) glycol | | | 2 |
| Total | 100mg | 100 mg | 100 mg |

| | | | |
|---|---|---|---|
| The amount blended of pemafibrate in the table is a value calculated from the amount added. | | | |

### [Production Examples 13 to 21]

Tablets containing the components in the amounts (mg) thereof per tablet shown in Tables 12 to 14 are conventionally produced through a direct powder compression method.

**[Table 12]**

| Components | Amount blended (mg) (per tablet) | | |
|---|---|---|---|
| | Production Example 13 | Production Example 14 | Production Example 15 |
| Pemafibrate | 0.1 | 0.4 | 0.1 |
| Lactose monohydrate | q.s. | q.s. | q.s. |
| Magnesium stearate | 1.2 | 1.2 | 1.2 |
| Ethyl acrylate/methyl methacrylate copolymer | 1 | | |
| Aminoalkyl methacrylate copolymer E | | 3 | |
| Ammonioalkyl methacrylate copolymer | | | 5 |
| Carboxyvinyl polymer | | | |
| Methacrylic acid copolymer S | | | |
| Methacrylic acid copolymer L | | | |
| Methacrylic acid copolymer LD | | | |
| Dried methacrylic acid copolymer LD | | | |
| Total | 100 mg | 100 mg | 100 mg |

| | | | |
|---|---|---|---|
| The amount blended of pemafibrate in the table is a value calculated from the amount added. | | | |

**[Table 13]**

| Components | Amount blended (mg) (per tablet) | | |
|---|---|---|---|
| | Production Example 16 | Production Example 17 | Production Example 18 |
| Pemafibrate | 0.8 | 0.2 | 0.4 |
| Lactose monohydrate | q.s. | q.s. | q.s. |
| Magnesium stearate | 1.2 | 1.2 | 1.2 |
| Ethyl acrylate/methyl methacrylate copolymer | | | |
| Aminoalkyl methacrylate copolymer E | | | |
| Ammonioalkyl methacrylate copolymer | | | |
| Carboxyvinyl polymer | 10 | | |
| Methacrylic acid copolymer S | | 20 | |
| Methacrylic acid copolymer L | | | 1 |
| Methacrylic acid copolymer LD | | | |
| Dried methacrylic acid copolymer LD | | | |
| Total | 100 mg | 100 mg | 100 mg |

| | | | |
|---|---|---|---|
| The amount blended of pemafibrate in the table is a value calculated from the amount added. | | | |

**[Table 14]**

| Components | Amount blended (mg) (per tablet) | | |
|---|---|---|---|
| | Production Example 19 | Production Example 20 | Production Example 21 |
| Pemafibrate | 0.1 | 0.4 | 0.1 |
| Lactose monohydrate | q.s. | q.s. | q.s. |
| Magnesium stearate | 1.2 | 1.2 | 1.2 |
| Ethyl acrylate/methyl methacrylate copolymer | | | 0.1 |
| Aminoalkyl methacrylate copolymer E | | | 0.2 |
| Ammonioalkyl methacrylate copolymer | | | 0.1 |
| Carboxyvinyl polymer | | | 0.2 |
| Methacrylic acid copolymer S | | | 0.1 |
| Methacrylic acid copolymer L | | | 0.2 |
| Methacrylic acid copolymer LD | | | 0.1 |
| Dried methacrylic acid copolymer LD | | | 0.2 |
| Total | 100 mg | 100 mg | 100 mg |

| | | | |
|---|---|---|---|
| The amount blended of pemafibrate in the table is a value calculated from the amount added. | | | |

### Industrial Applicability

The present invention enables provision of a pharmaceutical composition having excellent homogeneity and containing pemafibrate which exhibits plasma triglyceride concentration reducing action, HDL cholesterol increasing action, etc. The pharmaceutical composition can be used in, for example, pharmaceutical preparation industries.

## Claims

1. A pharmaceutical composition comprising the following components (A) and (B):
(A) pemafibrate, a salt thereof or a solvate thereof; and
(B) one or more selected from the group consisting of the following components (B-1) and (B-7):
(B-1) an alkylcellulose and a salt thereof; and
(B-7) a (meth)acrylic acid-based polymer.

2. The pharmaceutical composition according to claim 1, wherein the component (B) is an alkylcellulose or a salt thereof.

3. The pharmaceutical composition according to claim 1 or 2, wherein the component (B) is a C1-C6 alkylcellulose.

4. The pharmaceutical composition according to any one of claims 1 to 3, wherein the component (B) is one or more selected from the group consisting of methylcellulose and ethylcellulose.

5. The pharmaceutical composition according to claim 1, wherein the component (B) is a (meth)acrylic acid-based polymer.

6. The pharmaceutical composition according to claim 5, wherein the component (B) is a polymer derived from one or more monomers selected from the group consisting of acrylic acid, methacrylic acid, methyl methacrylate, ethyl acrylate, butyl methacrylate, dimethylaminoethyl methacrylate and trimethylammoniumethyl methacrylate chloride..

7. The pharmaceutical composition according to claim 5 or 6, wherein the component (B) is one or more selected from the group consisting of ethyl acrylate/methyl methacrylate copolymer, aminoalkyl methacrylate copolymer E, ammonioalkyl methacrylate copolymer, carboxyvinyl polymer, methacrylic acid copolymer S, methacrylic acid copolymer L and methacrylic acid copolymer LD.

8. The pharmaceutical composition according to any one of claims 1 to 7, wherein the pharmaceutical composition is a solid preparation.

9. The pharmaceutical composition according to any one of claims 1 to 8, wherein a dosage form thereof is a tablet, a capsule, a granule, a powder or a pill.

10. A method for improving content uniformity of pemafibrate, a salt thereof or a solvate thereof in a pharmaceutical composition, the method comprising the step of incorporating one or more selected from the group consisting of an alkylcellulose, a salt thereof and a (meth)acrylic acid-based polymer in a pharmaceutical composition comprising pemafibrate, a salt thereof or a solvate thereof.
